# EUROPEAN PATENT SPECIFICATION

(11) **EP 1 383 897 B1**
(45) Date of publication and mention of the grant of the patent: **28.06.2006**
(21) Application number: 02740565.3
(22) Date of filing: 03.05.2002
(51) Int. Cl.: C12N 15/52

(54) **SELF-CONTAINING LACTOCOCCUS STRAIN**
SELBSTERHALTENDER LACTOCOCCUS STAMM
SOUCHE DE LACTOCOCCUS A AUTO-CONFINEMENT

(30) Priority: 03.05.2001 EP 01201631; 07.12.2001 EP 01204785
(43) Date of publication of application: 28.01.2004
(73) Proprietor: Vlaams Interuniversitair Instituut voor Biotechnologie vzw., 9052 Zwijnaarde (BE)
(72) Inventor: STEIDLER, Lothar, Cork (IE)
(86) International application number: PCT/EP2002/004942
(87) International publication number: WO 2002/090551

(56) References cited:
- DE-A- 4 231 764
- STEIDLER L ET AL: "Treatment of murine colitis by Lactococcus lactis secreting interleukin-10" SCIENCE, AMERICAN ASSOCIATION FOR THE ADVANCEMENT OF SCIENCE,, US, vol. 289, no. 5483, 25 August 2000 (2000-08-25), pages 1352-1355, XP002208404 ISSN: 0036-8075 cited in the application
- TAYLOR G R ET AL: "MOLECULAR CHARACTERIZATION OF THE CELL CYCLE-REGULATED THYMIDYLATE SYNTHASE GENE OF SACCHAROMYCES-CEREVISIAE" JOURNAL OF BIOLOGICAL CHEMISTRY, vol. 262, no. 11, 1987, pages 5298-5307, XP001109399 ISSN: 0021-9258
- SCHOTTE L ET AL: "Secretion of biologically active murine interleukin-10 by Lactococcus lactis." ENZYME AND MICROBIAL TECHNOLOGY, vol. 27, no. 10, December 2000 (2000-12), pages 761-765, XP002225040 ISSN: 0141-0229
- ROSS P ET AL: "THYMIDYLATE SYNTHASE GENE FROM LACTOCOCCUS-LACTIS AS A GENETIC MARKER AN ALTERNATIVE TO ANTIBIOTIC RESISTANCE GENES" APPLIED AND ENVIRONMENTAL MICROBIOLOGY, vol. 56, no. 7, 1990, pages 2164-2169, XP008011631 ISSN: 0099-2240 cited in the application

### SEQUENCE LISTING

<110> VLAAMS INTERUNIVERSITAIR INSTITUUT VOOR BIOTECHNOLOGIE VZW
<120> SELF-CONTAINING LACTOCOCCUS STRAIN
<130> LS/ThyA/V085
<150> EP01201631.7
   <151> 2001-05-03
<150> EP01204785.8
   <151> 2001-12-07
<160> 26
<170> PatentIn version 3.1
<210> 1
   <211> 1000
   <212> DNA
   <213> Lactococcus lactis
<400> 1
   tatatacaat tgagcaaaag aaatttagtt attaaattac cagctggagt tcctccaatg      60
   gttgtagatt cactaagtcc agcaattatt tcaatggtga ttttctgttt gatgttcggg     120
   attcgtgtgg gattctctta tacgccattc catgatattt tcaatttctc aacacaacta     180
   attcaagcac cgttgactgg tgctgtggca aatccatggg ttcttatggg catctttacc     240
   tttggtaatt tcttatggtt ctttggtatc caccctaatt taattggggg aattttaaat     300
   ccattgttat taacaatgtc atatgctaat attgatgcct atgctgccgg aaaacctgta     360
   ccatacttac aaatgatgat tgtgtttgct gtgggtgcga acgcatgggg cggaagtgga     420
   aatacttatg ggttagttat ttcaatgttt acggcaaaat ctgaacgcta taaacaatta     480
   ttaaaattag gtgcaattcc tagtattttc aatatcagtg aaccattact ttttggtctt     540
   ccaatgatgt taaatcctct tttctttatt cctttggttt tccaaccagc aattttagga     600
   actgtagcat tgggcttggc aaagatatta tatattacaa atctgaatcc aatgacggca     660
   cttcttcctt ggacgacacc agcacctgtg agaatggcca tttcaggtgg acttccattt     720
   ttgattattt ttgcaatctg tttagtcttg aatgttctta tttactaccc attctttaag     780
   gtggcgtata ataaagcttt agaagaagaa aaagcagctg ttgaattaga gggttcagaa     840
   actgcctgat ggatattttt tataaatctg gtttgaacaa attatattga catctctttt     900
   tctatcctga taattctgag aggttatttt gggaaatact attgaaccat atcgaggtgt     960
   gtggtataat gaagggaatt aaaaaagata ggaaaatttc                          1000
<210> 2
   <211> 1000
   <212> DNA
   <213> Lactococcus lactis
<400> 2
   taaattaatc tataagttac tgacaaaact gtcagtaact ttttttgtgg gaaaaatgta      60
   tttttatgac cgtaaagaat ctgtcagtag aagtctgaaa ttcgtttaaa aatcgactag     120
   aataggcttt aacgacaaga tgttttaaag agtacgctct aaatgtattt ttgtattttt     180
   gtttgattac gaagtttaaa tttaattgac aaatgtttta aaatgagtat aataggactt     240
   gtaaccgatt ttatttttat aaaggagaaa gaaagatgaa caaactttta cttggaacag     300
   cctttatagg ggctagctta ctgattggtg ggggtgctca tgcagatcaa atgtttatcg     360
   tttgtataat cataatactg gtgagcactc tatacaacta gtgggacacc aaaagaatgc     420
   taatgtaagt gcgggttgga cttatgaagg tgtcggttgg atcgcaccaa caacaagttc     480
   aagcccagtt taccgtgtgt acaatccaaa tgcattatta cacaaaaagc aagtatgaag     540
   cccaaagttt agtaaataag ggttggaaat gggataataa cggaaaggcg gtcttctatt     600
   ctggaggttc tcaagccgta tatgtcgctt ataatcccaa tgcacaatct ggcgctcaca     660
   attacacgga aagtagcttt gagcaaaata gcttattgaa tactggttgg aaatatgggg     720
   cagtagcttg gtacgggatt ggagtaaaaa acgaaatgtt aaacattgct caaattgtta     780
   gtggtaattt ttctagtatt gttggaactt ggaaagatac ttctggaaat atgcttgaaa     840
   ttaatgcaat gggaaatctt actttaatat ggaaaggggc aaagaatcaa acctttgaac     900
   ttggcgcagg tcaacaattt aatggaactg cagatattgc cttaaaaaat ggagagattt     960
   cccctggtag tccacttaac atttttgttg taccaacaga                          1000
<210> 3
   <211> 7157
   <212> DNA
   <213> Lactococcus lactis
<220>
   <221> CDS
   <222> (4473)..(5312)
   <223>
<220>
   <221> misc_feature
   <222> (2)..(2)
   <223> 'n' may be any base
<220>
   <221> misc_feature
   <222> (5)..(5)
   <223> 'n' may be any base
<220>
   <221> misc_feature
   <222> (6612)..(6612)
   <223> 'n' may be any base
<220>
   <221> misc_feature
   <222> (7099)..(7099)
   <223> 'n' may be any base
<220>
   <221> misc_feature
   <222> (7110)..(7110)
   <223> 'n' may be any base
<220>
   <221> misc_feature
   <222> (7117)..(7141)
   <223> 'n' may be any base
<220>
   <221> misc_feature
   <222> (7143)..(7147)
   <223> 'n' may be any base
<220>
   <221> misc_feature
   <222> (7149)..(7156)
   <223> 'n' may be any base
<400> 3
   gnagnggttt tcccagtccg acgttgtaaa acgacggcca gtgaattcat taacagcctt 60
   ttgagcagct agctcattat tttgaaataa atcataaatt tctttcccac tatctgattt 120
   atgattgcta gcatatttgt tgtataatcg aacgagtcca ttttgaacag atccatatag 180
   attgagtgaa ctataaaata catctatatc atagttgagt ttgttcacaa tcatgagacc 240
   aaattctcca gcatttcgtg tagaaccacg ataaagctgt ttatttagca aaatggcacc 300
   tccgacacct gtacctaaag tcatgcaaat aaaattttgg ctttcttgtc cattccctag 360
   ccaaagttca gctagacctg cacaattggc atcattttca acataaaccg gaagatttaa 420
   atgtttttgt agttctgtcc ccaatggata gccataaaga tcagttagag ctcctgccag 480
   taataatgtt ccctttttgt cagaagttcc gggaacactt acaccaattg cagatactga 540
   atgatgagct tttaactgat gaatatttgt gagcaagcta tccataattt tttctttttt 600
   taatggggtt ggaacttgta aatgttgtat gatcgttcca tcactagtta caagaccaaa 660
   ttttataaat gtaccaccga tatcaattcc tattgaataa tgcatctttt attacctctt 720
   tctctaattt gttttagtat agcaaaatca aaaaattaat tatggtatgc attatagata 780
   tgttgtataa ttttcacaaa aacggagaaa actatgaaaa caatagaaca gctcatgata 840
   gattcagcag atttaatgtc agattttatt caattgacaa tttttatatt ccgcaaggag 900
   gattttcaac ttttttatag gagtgatgaa gaagagcaag ctttttcaag gtaatgactc 960
   caacttattg atagtgtttt atgttcagat aatgcccgat gactttgtca tgcagctcca 1020
   ccgattttga gaacgacagc gacttccgtc ccagccgtgc caggtgctgc ctcagattca 1080
   ggttatgccg ctcaattcgc tgcgtatatc gcttgctgat tacgtgcagc tttcccttca 1140
   ggcgggattc atacagcggc cagccatccg tcatccatat caccacgtca aagggtgaca 1200
   gcaggctcat aagacgcccc agcgtcgcca tagtgcgttc accgaatacg tgcgcaacaa 1260
   ccgtcttccg gagactgtca tacgcgtaaa acagccagcg ctggcgcgat ttagccccga 1320
   catagcccca ctgttcgtcc atttccgcgc agacgatgac gtcactgccc ggctgtatgc 1380
   gcgaggttac cgactgcggc ctgagttttt taagtgacgt aaaatcgtgt tgaggccaac 1440
   gcccataatg cgggctgttg cccggcatcc aacgccattc atggccatat caatgatttt 1500
   ctggtgcgta ccgggttgag aagcggtgta agtgaactgc agttgccatg ttttacggca 1560
   gtgagagcag agatagcgct gatgtccggc ggtgcttttg ccgttacgca ccaccccgtc 1620
   agtagctgaa caggagggac agctgataga aacagaagcc actggagcac ctcaaaaaca 1680
   ccatcataca ctaaatcagt aagttggcag catcacccct tttcaaaaga aatcatcgct 1740
   catttatctc agttgccctt gaaggaagag gtgaatttat tttatatgcc taagataaaa 1800
   ggatatatta cttatttttc tgtatttggt aaagaggagt atcttctact tatttttaaa 1860
   ggacaagaaa aacttgcaaa taatcctttc cccgttgaag taaaacaatt attaaaaagt 1920
   ggtattttac tctatcaaat gatttttcaa gaaaaattag attatgaaga attatttgag 1980
   aaaaatcagc atattatttc tccattgctt gctgctaaac caattgaatg gaatgattcc 2040
   aatacgtgag gaaagtaaat tcccataaaa catatctttt tgaaaaatat ttgggggaat 2100
   gtgttattcg tggagatgtt gcagagttaa aaaaagcttt ttcaaattat atgaataaag 2160
   gaactgctgg aaaattatct aataattcaa tgcgacataa gaaaaacatt ttgatttcag 2220
   tcatcactat gactactcgt tcggctatac agggaggatt acctgaagaa gaagcttttt 2280
   tgatgagtga tttatatatt caagagcttg aagaattaac ggaattagaa gaaattagaa 2340
   cgcttgccta taatgtgatg atcgattttg cagataaagt gaaacagcat cgatattgtc 2400
   aggtttctta taaaatatta tcttgtcaaa agtatattgt taatcattta tacgaaaaac 2460
   taagtgtgag tgaaattgca gaagagctac acatgaatat ttcttattta tcttcacaat 2520
   tcaaaaaaga gacagggcaa acaattacaa actttattca ggagaagcga atagaagaag 2580
   ctagagaatt aatccttttc tcagactatc ctttttcaag aatttatacc ttgttggttt 2640
   tactgccaaa gtcattttat aaaaatattt aaaaaatata ctggaataac tcccaaaaag 2700
   tttcaagatc agtatattta tcatgcctct acatcaatat atgattgaaa ttaaaaaaag 2760
   acctagaatt tcaaaattga taaaatacat acctaaaata ttaattctgt actattacgg 2820
   gtggagtatc tactgtataa tgagggtata aattatggaa gaagggagta aaactaaatt 2880
   tattgatggt tttacgaatt aattaggata ttttttttaa aaaccaaaga aaacgcttac 2940
   aaacgttaaa ggagtgaatc taaagatgga caaatttgaa aaatggctaa ataagacctt 3000
   gatgccactt gcctcaaaaa tgaataaaaa tcatttcatt tcggcattaa gtgaagcatt 3060
   tatgagatgt atgcccttaa cattagggat tgcattattg acaattatag gatactttcc 3120
   agttcctgcc tgggtagatt tcttaaactc tattggactg gctcagcatt tttcagcagt 3180
   tattggtgca gttaccagtg cgctagcaat ttatgtaact tataattttg cttattctta 3240
   tgtaaatcgt catgaatata atggccatac ggccggttta ttatcaatcg caagtttgtt 3300
   aatgctaatg ccacaaatta ttactgtccc tgtagtaaaa aacattccaa ccgaatttcc 3360
   gaaatccgcg gtagttgaca gtgtgtcaaa tgttgaagca tttcaaacgg tatacacggg 3420
   tagcacagga ttaattgtag caatcataat tggttttatt gtttcattag tctatataca 3480
   attgagcaaa agaaatttag ttattaaatt accagctgga gttcctccaa tggttgtaga 3540
   ttcactaagt ccagcaatta tttcaatggt gattttctgt ttgatgttcg ggattcgtgt 3600
   gggattctct tatacgccat tccatgatat tttcaatttc tcaacacaac taattcaagc 3660
   accgttgact ggtgctgtgg caaatccatg ggttcttatg ggcatcttta cctttggtaa 3720
   tttcttatgg ttctttggta tccaccctaa tttaattggg ggaattttaa atccattgtt 3780
   attaacaatg tcatatgcta atattgatgc ctatgctgcc ggaaaacctg taccatactt 3840
   acaaatgatg attgtgtttg ctgtgggtgc gaacgcatgg ggcggaagtg gaaatactta 3900
   tgggttagtt atttcaatgt ttacggcaaa atctgaacgc tataaacaat tattaaaatt 3960
   aggtgcaatt cctagtattt tcaatatcag tgaaccatta ctttttggtc ttccaatgat 4020
   gttaaatcct cttttcttta ttcctttggt tttccaacca gcaattttag gaactgtagc 4080
   attgggcttg gcaaagatat tatatattac aaatctgaat ccaatgacgg cacttcttcc 4140
   ttggacgaca ccagcacctg tgagaatggc catttcaggt ggacttccat ttttgattat 4200
   ttttgcaatc tgtttagtct tgaatgttct tatttactac ccattcttta aggtggcgta 4260
   taataaagct ttagaagaag aaaaagcagc tgttgaatta gagggttcag aaactgcctg 4320
   atggatattt tttataaatc tggtttgaac aaattatatt gacatctctt tttctatcct 4380
   gataattctg agaggttatt ttgggaaata ctattgaacc atatcgaggt gtgtggtata 4440
   atgaagggaa ttaaaaaaga taggaaaatt tc atg act tac gca gat caa gtt     4493
                                       Met Thr Tyr Ala Asp Gln Val
                                       1               5
   ttt aaa caa aat atc caa aat atc cta gat aat ggt gtt ttt tca gaa     4541
   Phe Lys Gln Asn Ile Gln Asn Ile Leu Asp Asn Gly Val Phe Ser Glu
           10                  15                  20
   aat gca aga cca aag tat aag gat ggt caa atg gcg aat agc aaa tat     4589
   Asn Ala Arg Pro Lys Tyr Lys Asp Gly Gln Met Ala Asn Ser Lys Tyr
       25                  30                  35
   gtc act ggt tca ttc gtt act tat gat ttg caa aag ggg gag ttt cca     4637
   Val Thr Gly Ser Phe Val Thr Tyr Asp Leu Gln Lys Gly Glu Phe Pro
   40                  45                  50                  55
   att acc act ttg cgt cca att cca atc aaa tct gct att aaa gaa ttg     4685
   Ile Thr Thr Leu Arg Pro Ile Pro Ile Lys Ser Ala Ile Lys Glu Leu
                   60                  65                  70
   atg tgg ata tac caa gac caa aca agt gaa ctt tct gtt ctc gaa gag     4733
   Met Trp Ile Tyr Gln Asp Gln Thr Ser Glu Leu Ser Val Leu Glu Glu
               75                  80                  85
   aag tat gga gtc aaa tac tgg gga gaa tgg gga att ggt gat ggt acg     4781
   Lys Tyr Gly Val Lys Tyr Trp Gly Glu Trp Gly Ile Gly Asp Gly Thr
           90                  95                  100
   att ggg caa cgt tat ggt gca aca gtc aaa aaa tat aat atc att ggt     4829
   Ile Gly Gln Arg Tyr Gly Ala Thr Val Lys Lys Tyr Asn Ile Ile Gly
       105                 110                 115
   aaa tta tta gaa ggc ttg gcc aaa aat cca tgg aat cgt cgt aat atc      4877
   Lys Leu Leu Glu Gly Leu Ala Lys Asn Pro Trp Asn Arg Arg Asn Ile
   120                 125                 130                 135
   atc aac ctt tgg cag tat gaa gat ttt gag gaa aca gaa ggt ctt tta      4925
   Ile Asn Leu Trp Gln Tyr Glu Asp Phe Glu Glu Thr Glu Gly Leu Leu
                   140                 145                 150
   cca tgt gct ttc caa acg atg ttt gat gtc cgt cga gaa aaa gat ggt      4973
   Pro Cys Ala Phe Gln Thr Met Phe Asp Val Arg Arg Glu Lys Asp Gly
               155                 160                 165
   cag att tat ttg gat gcc aca ctg att caa cgt tca aac gat atg ctt      5021
   Gln Ile Tyr Leu Asp Ala Thr Leu Ile Gln Arg Ser Asn Asp Met Leu
           170                 175                 180
   gta gcc cac cat atc aat gcg atg caa tat gtt gct ttg caa atg atg      5069
   Val Ala His His Ile Asn Ala Met Gln Tyr Val Ala Leu Gln Met Met
       185                 190                 195
   att gca aaa cat ttt tct tgg aaa gtt ggg aaa ttc ttt tat ttt gta      5117
   Ile Ala Lys His Phe Ser Trp Lys Val Gly Lys Phe Phe Tyr Phe Val
   200                 205                 210                 215
   aat aat tta cat att tat gat aat cag ttt gag cag gca aat gaa tta      5165
   Asn Asn Leu His Ile Tyr Asp Asn Gln Phe Glu Gln Ala Asn Glu Leu
                   220                 225                 230
   atg aag cga aca gct tct gaa aaa gaa cct cgt ttg gtc ctt aat gtt      5213
   Met Lys Arg Thr Ala Ser Glu Lys Glu Pro Arg Leu Val Leu Asn Val
               235                 240                 245
   cct gat ggt aca aac ttt ttc gat att aaa cct gaa gat ttt gaa ctt      5261
   Pro Asp Gly Thr Asn Phe Phe Asp Ile Lys Pro Glu Asp Phe Glu Leu
           250                 255                 260
   gtg gac tat gag cca gta aaa cct caa ttg aaa ttt gat tta gca att      5309
   Val Asp Tyr Glu Pro Val Lys Pro Gln Leu Lys Phe Asp Leu Ala Ile
       265                 270                 275
   taa attaatctat aagttactga caaaactgtc agtaactttt tttgtgggaa           5362
   aaatgtattt ttatgaccgt aaagaatctg tcagtagaag tctgaaattc gtttaaaaat    5422
   cgactagaat aggctttaac gacaagatgt tttaaagagt acgctctaaa tgtatttttg    5482
   tatttttgtt tgattacgaa gtttaaattt aattgacaaa tgttttaaaa tgagtataat    5542
   aggacttgta accgatttta tttttataaa ggagaaagaa agatgaacaa acttttactt    5602
   ggaacagcct ttataggggc tagcttactg attggtgggg gtgctcatgc agatcaaatg    5662
   tttatcgttt gtataatcat aatactggtg agcactctat acaactagtg ggacaccaaa    5722
   agaatgctaa tgtaagtgcg ggttggactt atgaaggtgt cggttggatc gcaccaacaa    5782
   caagttcaag cccagtttac cgtgtgtaca atccaaatgc attattacac aaaaagcaag    5842
   tatgaagccc aaagtttagt aaataagggt tggaaatggg ataataacgg aaaggcggtc    5902
   ttctattctg gaggttctca agccgtatat gtcgcttata atcccaatgc acaatctggc    5962
   gctcacaatt acacggaaag tagctttgag caaaatagct tattgaatac tggttggaaa    6022
   tatggggcag tagcttggta cgggattgga gtaaaaaacg aaatgttaaa cattgctcaa    6082
   attgttagtg gtaatttttc tagtattgtt ggaacttgga aagatacttc tggaaatatg    6142
   cttgaaatta atgcaatggg aaatcttact ttaatatgga aaggggcaaa gaatcaaacc   6202
   tttgaacttg gcgcaggtca acaatttaat ggaactgcag atattgcctt aaaaaatgga   6262
   gagatttccc ctggtagtcc acttaacatt tttgttgtac caacagaagt tgctttccct   6322
   aataataaaa aagtagacga ttcaactggg caacaacgaa tttttgtgaa ttattctggt   6382
   acaagccctc aaatggcgaa tagtatggca gcggtggctt tttttagagt tattccatga   6442
   ttatattaaa gttagaattg aataaaatgt attattaaaa agataatatt atatcacgac   6502
   aaggcgacat ctatcaactt taccactggt atggaagtga ccattattac atcaggaaac   6562
   gctaaaacgg ttgtttttac acccgtaaaa taaataataa aataatgtgn aattactgac   6622
   agcattttgt cagtaatttt ttttatcaaa atcacacaaa aatgttcgtt gacgaacaaa   6682
   aaaaactatg ttataataat tcgtatgcga actaaaaaag aagcgattgg ccgactttta   6742
   aaagtagcca gcaaccaaat gtctcgagaa tttgataatt ttgcagctca acttgatttg   6802
   acaggtcagc aaatgtcaat tttagatttt cttggaaatc aaagcgaaga aggttcagga   6862
   aaagaaatta gtcagacgat gattgaatta gaatttaata tccgacgttc aacaacgacg   6922
   gaaattttac agcgcatgga aaagcggctt ttaattaatc gaagaacaag cctgaccgat   6982
   gcccgccaaa aatcagttga attaactgaa gaagggaaaa gatatttacc tgaaatcagg   7042
   gcttatatcc aagcacataa taaaaaagct tggcgtaatc atggtcatag ctgtttncct   7102
   ggttaggngg gccannnnnn nnnnnnnnnn nnncnnnnnc nnnnncnnnn cnnnc        7157
<210> 4
   <211> 279
   <212> PRT
   <213> Lactococcus lactis
<220>
   <221> misc_feature
   <222> (2)..(2)
   <223> 'n' may be any base
<220>
   <221> misc_feature
   <222> (5)..(5)
   <223> 'n' may be any base
<220>
   <221> misc_feature
   <222> (6612)..(6612)
   <223> 'n' may be any base
<220>
   <221> misc_feature
   <222> (7099)..(7099)
   <223> 'n' may be any base
<220>
   <221> misc_feature
   <222> (7110)..(7110)
   <223> 'n' may be any base
<220>
   <221> misc_feature
   <222> (7117)..(7141)
   <223> 'n' may be any base
<220>
   <221> misc_feature
   <222> (7143)..(7147)
   <223> 'n' may be any base
<220>
   <221> misc_feature
   <222> (7149)..(7156)
   <223> 'n' may be any base
<400> 4
   Met Thr Tyr Ala Asp Gln Val Phe Lys Gln Asn Ile Gln Asn Ile Leu
   1               5                   10                  15
   Asp Asn Gly Val Phe Ser Glu Asn Ala Arg Pro Lys Tyr Lys Asp Gly
               20                  25                  30
   Gln Met Ala Asn Ser Lys Tyr Val Thr Gly Ser Phe Val Thr Tyr Asp
           35                  40                  45
   Leu Gln Lys Gly Glu Phe Pro Ile Thr Thr Leu Arg Pro Ile Pro Ile
       50                  55                  60
   Lys Ser Ala Ile Lys Glu Leu Met Trp Ile Tyr Gln Asp Gln Thr Ser
   65                  70                  75                  80
   Glu Leu Ser Val Leu Glu Glu Lys Tyr Gly Val Lys Tyr Trp Gly Glu
                   85                  90                  95
   Trp Gly Ile Gly Asp Gly Thr Ile Gly Gln Arg Tyr Gly Ala Thr Val
               100                 105                 110
   Lys Lys Tyr Asn Ile Ile Gly Lys Leu Leu Glu Gly Leu Ala Lys Asn
           115                 120                 125
   Pro Trp Asn Arg Arg Asn Ile Ile Asn Leu Trp Gln Tyr Glu Asp Phe
       130                 135                 140
   Glu Glu Thr Glu Gly Leu Leu Pro Cys Ala Phe Gln Thr Met Phe Asp
   145                 150                 155                 160
   Val Arg Arg Glu Lys Asp Gly Gln Ile Tyr Leu Asp Ala Thr Leu Ile
                   165                 170                 175
   Gln Arg Ser Asn Asp Met Leu Val Ala His His Ile Asn Ala Met Gln
               180                 185                 190
   Tyr Val Ala Leu Gln Met Met Ile Ala Lys His Phe Ser Trp Lys Val
           195                 200                 205
   Gly Lys Phe Phe Tyr Phe Val Asn Asn Leu His Ile Tyr Asp Asn Gln
       210                 215                 220
   Phe Glu Gln Ala Asn Glu Leu Met Lys Arg Thr Ala Ser Glu Lys Glu
   225                 230                 235                 240
   Pro Arg Leu Val Leu Asn Val Pro Asp Gly Thr Asn Phe Phe Asp Ile
                   245                 250                 255
   Lys Pro Glu Asp Phe Glu Leu Val Asp Tyr Glu Pro Val Lys Pro Gln
               260                 265                 270
   Leu Lys Phe Asp Leu Ala Ile
           275
<210> 5
   <211> 7094
   <212> DNA
   <213> Lactococcus lactis
<220>
   <221> CDS
   <222> (4469)..(5305)
   <223>
<400> 5
   ggttttccca gtccgacgtt gtaaaacgac ggccagtgaa ttcattaaca gccttttgag      60
   cagctagctc attattttga aataaatcat aaatttcttt cccactatct gatttatgat     120
   tgctagcata tttgttgtat aatcgaacga gtccattttg aacagatcca tatagattga     180
   gtgaactata aaatacatct atatcatagt tgagtttgtt cacaatcatg agaccaaatt     240
   ctccagcatt tcgtgtagaa ccacgataaa gctgtttatt tagcaaaatg gcacctccga     300
   cacctgtacc taaagtcatg caaataaaat tttggctttc ttgtccattc cctagccaaa     360
   gttcagctag acctgcacaa ttggcatcat tttcaacata aaccggaaga tttaaatgtt     420
   tttgtagttc tgtccccaat ggatagccat aaagatcagt tagagctcct gccagtaata     480
   atgttccctt tttgtcagaa gttccgggaa cacttacacc aattgcagat actgaatgat     540
   gagcttttaa ctgatgaata tttgtgagca agctatccat aatttttctt ttttttaatg     600
   gggttggaac ttgtaaatgt tgtatgatcg ttccatcact agttacaaga ccaaatttta     660
   taaatgtacc accgatatca attcctattg aataatgcat cttttattac ctctttctct     720
   aatttgtttt agtatagcaa aatcaaaaaa ttaattatgg tatgcattat agatatgttg     780
   tataattttc acaaaaacgg agaaaactat gaaaacaata gaacagctca tgatagattc     840
   agcagattta atgtcagatt ttattcaatt gacaattttt atattccgca aggaggattt     900
   tcaacttttt tataggagtg atgaagaaga gcaagctttt tcaaggtaat gactccaact     960
   tattgatagt gttttatgtt cagataatgc ccgatgactt tgtcatgcag ctccaccgat    1020
   tttgagaacg acagcgactt ccgtcccagc cgtgccaggt gctgcctcag attcaggtta    1080
   tgccgctcaa ttcgctgcgt atatcgcttg ctgattacgt gcagctttcc cttcaggcgg    1140
   gattcataca gcggccagcc atccgtcatc catatcacca cgtcaaaggg tgacagcagg 1200
   ctcataagac gccccagcgt cgccatagtg cgttcaccga atacgtgcgc aacaaccgtc 1260
   ttccggagac tgtcatacgc gtaaaacagc cagcgctggc gcgatttagc cccgacatag 1320
   ccccactgtt cgtccatttc cgcgcagacg atgacgtcac tgcccggctg tatgcgcgag 1380
   gttaccgact gcggcctgag ttttttaagt gacgtaaaat cgtgttgagg ccaacgccca 1440
   taatgcgggc tgttgcccgg catccaacgc cattcatggc catatcaatg attttctggt 1500
   gcgtaccggg ttgagaagcg gtgtaagtga actgcagttg ccatgtttta cggcagtgag 1560
   agcagagata gcgctgatgt ccggcggtgc ttttgccgtt acgcaccacc ccgtcagtag 1620
   ctgaacagga gggacagctg atagaaacag aagccactgg agcacctcaa aaacaccatc 1680
   atacactaaa tcagtaagtt ggcagcatca ccctttttca aaagaaatca tcgctcattt 1740
   atctcagttg cccttgaagg aagaggtgaa tttattttat atgcctaaga taaaaggata 1800
   tattacttat ttttctgtat ttggtaaaga ggagtatctt ctacttattt ttaaaggaca 1860
   agaaaaactt gcaaataatc ctttccccgt tgaagtaaaa caattattaa aaagtggtat 1920
   tttactctat caaatgattt ttcaagaaaa attagattat gaagaattat ttgagaaaaa 1980
   tcagcatatt atttctccat tgcttgctgc taaaccaatt gaatggaatg attccaatac 2040
   gtgaggaaag taaattccca taaaacatat ctttttgaaa aatatttggg ggaatgtgtt 2100
   attcgtggag atgttgcaga gttaaaaaaa gctttttcaa attatatgaa taaaggaact 2160
   gctggaaaat tatctaataa ttcaatgcga cataagaaaa acattttgat ttcagtcatc 2220
   actatgacta ctcgttcggc tatacaggga ggattacctg aagaagaagc ttttttgatg 2280
   agtgatttat atattcaaga gcttgaagaa ttaacggaat tagaagaaat tagaacgctt 2340
   gcctataatg tgatgatcga ttttgcagat aaagtgaaac agcatcgata ttgtcaggtt 2400
   tcttataaaa tattatcttg tcaaaagtat attgttaatc atttatacga aaaactaagt 2460
   gtgagtgaaa ttgcagaaga gctacacatg aatatttctt atttatcttc acaattcaaa 2520
   aaagagacag ggcaaacaat tacaaacttt attcaggaga agcgaataga agaagctaga 2580
   gaattaatcc ttttctcaga ctatcctttt tcaagaattt ataccttgtt ggttttactg 2640
   ccaaagtcat tttataaaaa tatttaaaaa atatactgga ataactccca aaaagtttca 2700
   agatcagtat atttatcatg cctctacatc aatatatgat tgaaattaaa aaaagaccta 2760
   gaatttcaaa attgataaaa tacataccta aaatattaat tctgtactat tacgggtgga 2820
   gtatctactg tataatgagg gtataaatta tggaagaagg gagtaaaact aaatttattg 2880
   atggttttac gaattaatta ggatattttt tttaaaaacc aaagaaaacg cttacaaacg 2940
   ttaaaggagt gaatctaaag atggacaaat ttgaaaaatg gctaaataag accttgatgc 3000
   cacttgcctc aaaaatgaat aaaaatcatt tcatttcggc attaagtgaa gcatttatga 3060
   gatgtatgcc cttaacatta gggattgcat tattgacaat tataggatac tttccagttc 3120
   ctgcctgggt agatttctta aactctattg gactggctca gcatttttca gcagttattg 3180
   gtgcagttac cagtgcgcta gcaatttatg taacttataa ttttgcttat tcttatgtaa   3240
   atcgtcatga atataatggc catacggccg gtttattatc aatcgcaagt ttgttaatgc   3300
   taatgccaca aattattact gtccctgtag taaaaaacat tccaaccgaa tttccgaaat   3360
   ccgcggtagt tgacagtgtg tcaaatgttg aagcatttca aacggtatac acgggtagca   3420
   caggattaat tgtagcaatc ataattggtt ttattgtttc attagtctat atacaattga   3480
   gcaaaagaaa tttagttatt aaattaccag ctggagttcc tccaatggtt gtagattcac   3540
   taagtccagc aattatttca atggtgattt tctgtttgat gttcgggatt cgtgtgggat   3600
   tctcttatac gccattccat gatattttca atttctcaac acaactaatt caagcaccgt   3660
   tgactggtgc tgtggcaaat ccatgggttc ttatgggcat ctttaccttt ggtaatttct   3720
   tatggttctt tggtatccac cctaatttaa ttgggggaat tttaaatcca ttgttattaa   3780
   caatgtcata tgctaatatt gatgcctatg ctgccggaaa acctgtacca tacttacaaa   3840
   tgatgattgt gtttgctgtg ggtgcgaacg catggggcgg aagtggaaat acttatgggt   3900
   tagttatttc aatgtttacg gcaaaatctg aacgctataa acaattatta aaattaggtg   3960
   caattcctag tattttcaat atcagtgaac cattactttt tggtcttcca atgatgttaa   4020
   atcctctttt ctttattcct ttggttttcc aaccagcaat tttaggaact gtagcattgg   4080
   gcttggcaaa gatattatat attacaaatc tgaatccaat gacggcactt cttccttgga   4140
   cgacaccagc acctgtgaga atggccattt caggtggact tccatttttg attatttttg   4200
   caatctgttt agtcttgaat gttcttattt actacccatt ctttaaggtg gcgtataata   4260
   aagctttaga agaagaaaaa gcagctgttg aattagaggg ttcagaaact gcctgatgga   4320
   tattttttat aaatctggtt tgaacaaatt atattgacat ctctttttct atcctgataa   4380
   ttctgagagg ttattttggg aaatactatt gaaccatatc gaggtggtgt ggtataatga   4440
   agggaattaa aaaagatagg aaaatttc atg act tac gca gat caa gtt ttt      4492
                                   Met Thr Tyr Ala Asp Gln Val Phe
                                   1               5
   aaa caa aat atc caa aat atc cta gat aat ggt gtt ttt tca gaa aat     4540
   Lys Gln Asn Ile Gln Asn Ile Leu Asp Asn Gly Val Phe Ser Glu Asn
       10                  15                  20
   gca aga cca aag tat aag gat ggt caa atg gcg aat agc aaa tat gtc     4588
   Ala Arg Pro Lys Tyr Lys Asp Gly Gln Met Ala Asn Ser Lys Tyr Val
   25                  30                  35                  40
   act ggt tca ttc gtt act tat gat ttg caa aag ggg gag ttt cca att     4636
   Thr Gly Ser Phe Val Thr Tyr Asp Leu Gln Lys Gly Glu Phe Pro Ile
                   45                  50                  55
   acc act ttg cgt cca att cca atc aaa tct gct att aaa gaa ttg atg     4684
   Thr Thr Leu Arg Pro Ile Pro Ile Lys Ser Ala Ile Lys Glu Leu Met
               60                  65                  70
   tgg ata tac caa gac caa aca agt gaa ctt tct gtt ctc gaa gag aag     4732
   Trp Ile Tyr Gln Asp Gln Thr Ser Glu Leu Ser Val Leu Glu Glu Lys
           75                  80                  85
   tat gga gtc aaa tac tgg gga gaa tgg gga att ggt gat ggt acg att     4780
   Tyr Gly Val Lys Tyr Trp Gly Glu Trp Gly Ile Gly Asp Gly Thr Ile
       90                  95                  100
   ggg caa cgt tat ggt gca aca gtc aaa aaa tat aat atc att ggt aaa      4828
   Gly Gln Arg Tyr Gly Ala Thr Val Lys Lys Tyr Asn Ile Ile Gly Lys
   105                 110                 115                 120
   tta tta gaa ggc ttg gcc aaa aat cca tgg aat cgt cgt aat atc atc      4876
   Leu Leu Glu Gly Leu Ala Lys Asn Pro Trp Asn Arg Arg Asn Ile Ile
                   125                 130                 135
   aac ctt tgg cag tat gaa gat ttt gag gaa aca gaa ggt ctt tta cca      4924
   Asn Leu Trp Gln Tyr Glu Asp Phe Glu Glu Thr Glu Gly Leu Leu Pro
               140                 145                 150
   tgt gct ttc caa acg atg ttt gat gtc cgt cga gaa aaa gat ggt cag      4972
   Cys Ala Phe Gln Thr Met Phe Asp Val Arg Arg Glu Lys Asp Gly Gln
           155                 160                 165
   att tat ttg gat gcc aca ctg att caa cgt tca aac gat atg ctt gta      5020
   Ile Tyr Leu Asp Ala Thr Leu Ile Gln Arg Ser Asn Asp Met Leu Val
       170                 175                 180
   gcc cac cat atc aat gcg atg caa tat gtt gct ttg caa atg atg att      5068
   Ala His His Ile Asn Ala Met Gln Tyr Val Ala Leu Gln Met Met Ile
   185                 190                 195                 200
   gca aaa cat ttt tct tgg aaa gtt ggg aaa ttc ttt tat ttt gta aat      5116
   Ala Lys His Phe Ser Trp Lys Val Gly Lys Phe Phe Tyr Phe Val Asn
                   205                 210                 215
   aat tta cat att tat gat aat cag ttt gag cag gca aat gaa tta atg      5164
   Asn Leu His Ile Tyr Asp Asn Gln Phe Glu Gln Ala Asn Glu Leu Met
               220                 225                 230
   aag cga aca gct tct gaa aaa gaa cct cgt ttg gtc ctt aat gtt cct      5212
   Lys Arg Thr Ala Ser Glu Lys Glu Pro Arg Leu Val Leu Asn Val Pro
           235                 240                 245
   gat ggt aca aac ttt ttc gat att aaa cct gaa gat ttt gaa ctt gtg      5260
   Asp Gly Thr Asn Phe Phe Asp Ile Lys Pro Glu Asp Phe Glu Leu Val
       250                 255                 260
   gac tat gag cca gta aaa cct caa ttg aaa ttt gat tta gca att          5305
   Asp Tyr Glu Pro Val Lys Pro Gln Leu Lys Phe Asp Leu Ala Ile
   265                 270                 275
   taaattaatc tataagttac tgacaaaact gtcagtaact ttttttgtgg gaaaaatgta    5365
   tttttatgac cgtaaagaat ctgtcagtag aagtctgaaa ttcgtttaaa aatcgactag    5425
   aataggcttt aacgacaaga tgttttaaag agtacgctct aaatgtattt ttgtattttt    5485
   gtttgattac gaagtttaaa tttaattgac aaatgtttta aaatgagtat aataggactt    5545
   gtaaccgatt ttatttttat aaaggagaaa gaaagatgaa caaactttta cttggaacag    5605
   cctttatagg ggctagctta ctgattggtg ggggtgctca tgcagatcaa atgtttatcg    5665
   tttgtataat cataatactg gtgagcactc tatacaacta gtgggacacc aaaagaatgc    5725
   taatgtaagt gcgggttgga cttatgaagg tgtcggttgg atcgcaccaa caacaagttc    5785
   aagcccagtt taccgtgtgt acaatccaaa tgcattatta cacaaaaagc aagtatgaag    5845
   cccaaagttt agtaaataag ggttggaaat gggataataa cggaaaggcg gtcttctatt    5905
   ctggaggttc tcaagccgta tatgtcgctt ataatcccaa tgcacaatct ggcgctcaca    5965
   attacacgga aagtagcttt gagcaaaata gcttattgaa tactggttgg aaatatgggg 6025
   cagtagcttg gtacgggatt ggagtaaaaa acgaaatgtt aaacattgct caaattgtta 6085
   gtggtaattt ttctagtatt gttggaactt ggaaagatac ttctggaaat atgcttgaaa 6145
   ttaatgcaat gggaaatctt actttaatat ggaaaggggc aaagaatcaa acctttgaac 6205
   ttggcgcagg tcaacaattt aatggaactg cagatattgc cttaaaaaat ggagagattt 6265
   cccctggtag tccacttaac atttttgttg taccaacaga agttgctttc cctaataata 6325
   aaaaagtaga cgattcaact gggcaacaac gaatttttgt gaattattct ggtacaagcc 6385
   ctcaaatggc gaatagtatg gcagcggtgg ctttttttag agttattcca tgattatatt 6445
   aaagttagaa ttgaataaaa tgtattatta aaaagataat attatatcac gacaaggcga 6505
   catctatcaa ctttaccact ggtatggaag tgaccattat tacatcagga aacgctaaaa 6565
   cggttgtttt tacacccgta aaataaataa taaaataatg tgaaattact gacagcattt 6625
   tgtcagtaat tttttttatc aaaatcacac aaaaatgttc gttgacgaac aaaaaaaact 6685
   atgttataat aattcgtatg cgaactaaaa aagaagcgat tggccgactt ttaaaagtag 6745
   ccagcaacca aatgtctcga gaatttgata attttgcagc tcaacttgat ttgacaggtc 6805
   agcaaatgtc aattttagat tttcttggaa atcaaagcga agaaggttca ggaaaagaaa 6865
   ttagtcagac gatgattgaa ttagaattta atatccgacg ttcaacaacg acggaaattt 6925
   tacagcgcat ggaaaagcgg cttttaatta atcgaagaac aagcctgacc gatgcccgcc 6985
   aaaaatcagt tgaattaact gaagaaggga aaagatattt acctgaaatc agggcttata 7045
   tccaagcaca taataaaaaa gcttggcgta atcatggtca tagctgttt 7094
<210> 6
   <211> 279
   <212> PRT
   <213> Lactococcus lactis
<400> 6
   Met Thr Tyr Ala Asp Gln Val Phe Lys Gln Asn Ile Gln Asn Ile Leu
   1               5                   10                  15
   Asp Asn Gly Val Phe Ser Glu Asn Ala Arg Pro Lys Tyr Lys Asp Gly
               20                  25                  30
   Gln Met Ala Asn Ser Lys Tyr Val Thr Gly Ser Phe Val Thr Tyr Asp
           35                  40                  45
   Leu Gln Lys Gly Glu Phe Pro Ile Thr Thr Leu Arg Pro Ile Pro Ile
       50                  55                  60
   Lys Ser Ala Ile Lys Glu Leu Met Trp Ile Tyr Gln Asp Gln Thr Ser
   65                  70                  75                  80
   Glu Leu Ser Val Leu Glu Glu Lys Tyr Gly Val Lys Tyr Trp Gly Glu
                   85                  90                  95
   Trp Gly Ile Gly Asp Gly Thr Ile Gly Gln Arg Tyr Gly Ala Thr Val
               100                 105                 110
   Lys Lys Tyr Asn Ile Ile Gly Lys Leu Leu Glu Gly Leu Ala Lys Asn
           115                 120                 125
   Pro Trp Asn Arg Arg Asn Ile Ile Asn Leu Trp Gln Tyr Glu Asp Phe
       130                 135                 140
   Glu Glu Thr Glu Gly Leu Leu Pro Cys Ala Phe Gln Thr Met Phe Asp
   145                 150                 155                 160
   Val Arg Arg Glu Lys Asp Gly Gln Ile Tyr Leu Asp Ala Thr Leu Ile
                   165                 170                 175
   Gln Arg Ser Asn Asp Met Leu Val Ala His His Ile Asn Ala Met Gln
               180                 185                 190
   Tyr Val Ala Leu Gln Met Met Ile Ala Lys His Phe Ser Trp Lys Val
           195                 200                 205
   Gly Lys Phe Phe Tyr Phe Val Asn Asn Leu His Ile Tyr Asp Asn Gln
       210                 215                 220
   Phe Glu Gln Ala Asn Glu Leu Met Lys Arg Thr Ala Ser Glu Lys Glu
   225                 230                 235                 240
   Pro Arg Leu Val Leu Asn Val Pro Asp Gly Thr Asn Phe Phe Asp Ile
                   245                 250                 255
   Lys Pro Glu Asp Phe Glu Leu Val Asp Tyr Glu Pro Val Lys Pro Gln
               260                 265                 270
   Leu Lys Phe Asp Leu Ala Ile
           275
<210> 7
   <211> 1000
   <212> DNA
   <213> Lactococcus lactis
<400> 7
   atatacaatt gagcaaaaga aatttagtta ttaaattacc agctggagtt cctccaatgg 60
   ttgtagattc actaagtcca gcaattattt caatggtgat tttctgtttg atgttcggga 120
   ttcgtgtggg attctcttat acgccattcc atgatatttt caatttctca acacaactaa 180
   ttcaagcacc gttgactggt gctgtggcaa atccatgggt tcttatgggc atctttacct 240
   ttggtaattt cttatggttc tttggtatcc accctaattt aattggggga attttaaatc 300
   cattgttatt aacaatgtca tatgctaata ttgatgccta tgctgccgga aaacctgtac 360
   catacttaca aatgatgatt gtgtttgctg tgggtgcgaa cgcatggggc ggaagtggaa 420
   atacttatgg gttagttatt tcaatgttta cggcaaaatc tgaacgctat aaacaattat 480
   taaaattagg tgcaattcct agtattttca atatcagtga accattactt tttggtcttc 540
   caatgatgtt aaatcctctt ttctttattc ctttggtttt ccaaccagca attttaggaa 600
   ctgtagcatt gggcttggca aagatattat atattacaaa tctgaatcca atgacggcac 660
   ttcttccttg gacgacacca gcacctgtga gaatggccat ttcaggtgga cttccatttt 720
   tgattatttt tgcaatctgt ttagtcttga atgttcttat ttactaccca ttctttaagg 780
   tggcgtataa taaagcttta gaagaagaaa aagcagctgt tgaattagag ggttcagaaa 840
   ctgcctgatg gatatttttt ataaatctgg tttgaacaaa ttatattgac atctcttttt 900
   ctatcctgat aattctgaga ggttattttg ggaaatacta ttgaaccata tcgaggtggt 960
   gtggtataat gaagggaatt aaaaaagata ggaaaatttc 1000
<210> 8
   <211> 24
   <212> DNA
   <213> Artificial Sequence
<220>
   <223> oligonucleotide primer
<400> 8
   atgacttacg cagatcaagt tttt 24
<210> 9
   <211> 27
   <212> DNA
   <213> Artificial Sequence
<220>
   <223> oligonucleotide primer
<400> 9
   ttaaattgct aaatcaaatt tcaattg 27
<210> 10
   <211> 20
   <212> DNA
   <213> Artificial Sequence
<220>
   <223> oligonucleotide primer
<400> 10
   tctgattgag taccttgacc 20
<210> 11
   <211> 22
   <212> DNA
   <213> Artificial Sequence
<220>
   <223> oligonucleotide primer
<400> 11
   gcaatcataa ttggttttat tg 22
<210> 12
   <211> 22
   <212> DNA
   <213> Artificial Sequence
<220>
   <223> oligonucleotide primer
<400> 12
   cttacatgac tatgaaaatc cg 22
<210> 13
   <211> 23
   <212> DNA
   <213> Artificial Sequence
<220>
   <223> oligonucleotide primer
<400> 13
   cttttttatt attagggaaa gca 23
<210> 14
   <211> 21
   <212> DNA
   <213> Artificial Sequence
<220>
   <223> expression unit comprising the lactococcal P1 promoter, the E.col i bacteriophage T7 expression signals, putative RNA stabilising sequence and modified gene10 ribosomal binding site
<400> 14
   gattaagtca tcttacctct t 21
<210> 15
   <211> 39
   <212> DNA
   <213> Artificial Sequence
<220>
   <223> thyA-, P1-T7-usp45-hIL10
<400> 15
   agataggaaa atttcatgga ttaagtcatc ttacctctt 39
<210> 16
   <211> 36
   <212> DNA
   <213> Artificial Sequence
<220>
   <223> ATG not included, thyA-, P1-T7-usp45-hIL10
<400> 16
   agataggaaa atttcgatta agtcatctta cctctt 36
<210> 17
   <211> 48
   <212> DNA
   <213> Artificial Sequence
<220>
   <223> thyA promoter not included, theA-, P1-T7-usp45-hIL10
<400> 17
   tctgagaggt tattttggga aatactagat taagtcatct tacctctt 48
<210> 18
   <211> 40
   <212> DNA
   <213> Artificial Sequence
<220>
   <223> thyA-, usp45-hIL10
<400> 18
   aaaatccgta actaactaga attaatctat aagttactga 40
<210> 19
   <211> 6967
   <212> DNA
   <213> Lactococcus lactis
<220>
   <221> misc feature
   <223> Thy11̅
<400> 19
   attaacagcc ttttgagcag ctagctcatt attttgaaat aaatcataaa tttctttccc     60
   actatctgat ttatgattgc tagcatattt gttgtataat cgaacgagtc cattttgaac    120
   agatccatat agattgagtg aactataaaa tacatctata tcatagttga gtttgttcac    180
   aatcatgaga ccaaattctc cagcatttcg tgtagaacca cgataaagct gtttatttag    240
   caaaatggca cctccgacac ctgtacctaa agtcatgcaa ataaaatttt ggctttcttg    300
   tccattccct agccaaagtt cagctagacc tgcacaattg gcatcatttt caacataaac    360
   cggaagattt aaatgttttt gtagttctgt ccccaatgga tagccataaa gatcagttag    420
   agctcctgcc agtaataatg ttcccttttt gtcagaagtt ccgggaacac ttacaccaat    480
   tgcagatact gaatgatgag cttttaactg atgaatattt gtgagcaagc tatccataat    540
   tttttctttt tttaatgggg ttggaacttg taaatgttgt atgatcgttc catcactagt    600
   tacaagacca aattttataa atgtaccacc gatatcaatt cctattgaat aatgcatctt    660
   ttattacctc tttctctaat ttgttttagt atagcaaaat caaaaaatta attatggtat    720
   gcattataga tatgttgtat aattttcaca aaaacggaga aaactatgaa aacaatagaa    780
   cagctcatga tagattcagc agatttaatg tcagatttta ttcaattgac aatttttata    840
   ttccgcaagg aggattttca acttttttat aggagtgatg aagaagagca agctttttca    900
   aggtaatgac tccaacttat tgatagtgtt ttatgttcag ataatgcccg atgactttgt    960
   catgcagctc caccgatttt gagaacgaca gcgacttccg tcccagccgt gccaggtgct   1020
   gcctcagatt caggttatgc cgctcaattc gctgcgtata tcgcttgctg attacgtgca   1080
   gctttccctt caggcgggat tcatacagcg gccagccatc cgtcatccat atcaccacgt 1140
   caaagggtga cagcaggctc ataagacgcc ccagcgtcgc catagtgcgt tcaccgaata 1200
   cgtgcgcaac aaccgtcttc cggagactgt catacgcgta aaacagccag cgctggcgcg 1260
   atttagcccc gacatagccc cactgttcgt ccatttccgc gcagacgatg acgtcactgc 1320
   ccggctgtat gcgcgaggtt accgactgcg gcctgagttt tttaagtgac gtaaaatcgt 1380
   gttgaggcca acgcccataa tgcgggctgt tgcccggcat ccaacgccat tcatggccat 1440
   atcaatgatt ttctggtgcg taccgggttg agaagcggtg taagtgaact gcagttgcca 1500
   tgttttacgg cagtgagagc agagatagcg ctgatgtccg gcggtgcttt tgccgttacg 1560
   caccaccccg tcagtagctg aacaggaggg acagctgata gaaacagaag ccactggagc 1620
   acctcaaaaa caccatcata cactaaatca gtaagttggc agcatcaccc tttttcaaaa 1680
   gaaatcatcg ctcatttatc tcagttgccc ttgaaggaag aggtgaattt attttatatg 1740
   cctaagataa aaggatatat tacttatttt tctgtatttg gtaaagagga gtatcttcta 1800
   cttattttta aaggacaaga aaaacttgca aataatcctt tccccgttga agtaaaacaa 1860
   ttattaaaaa gtggtatttt actctatcaa atgatttttc aagaaaaatt agattatgaa 1920
   gaattatttg agaaaaatca gcatattatt tctccattgc ttgctgctaa accaattgaa 1980
   tggaatgatt ccaatacgtg aggaaagtaa attcccataa aacatatctt tttgaaaaat 2040
   atttggggga atgtgttatt cgtggagatg ttgcagagtt aaaaaaagct ttttcaaatt 2100
   atatgaataa aggaactgct ggaaaattat ctaataattc aatgcgacat aagaaaaaca 2160
   ttttgatttc agtcatcact atgactactc gttcggctat acagggagga ttacctgaag 2220
   aagaagcttt tttgatgagt gatttatata ttcaagagct tgaagaatta acggaattag 2280
   aagaaattag aacgcttgcc tataatgtga tgatcgattt tgcagataaa gtgaaacagc 2340
   atcgatattg tcaggtttct tataaaatat tatcttgtca aaagtatatt gttaatcatt 2400
   tatacgaaaa actaagtgtg agtgaaattg cagaagagct acacatgaat atttcttatt 2460
   tatcttcaca attcaaaaaa gagacagggc aaacaattac aaactttatt caggagaagc 2520
   gaatagaaga agctagagaa ttaatccttt tctcagacta tcctttttca agaatttata 2580
   ccttgttggt tttactgcca aagtcatttt ataaaaatat ttaaaaaata tactggaata 2640
   actcccaaaa agtttcaaga tcagtatatt tatcatgcct ctacatcaat atatgattga 2700
   aattaaaaaa agacctagaa tttcaaaatt gataaaatac atacctaaaa tattaattct 2760
   gtactattac gggtggagta tctactgtat aatgagggta taaattatgg aagaagggag 2820
   taaaactaaa tttattgatg gttttacgaa ttaattagga tatttttttt aaaaaccaaa 2880
   gaaaacgctt acaaacgtta aaggagtgaa tctaaagatg gacaaatttg aaaaatggct 2940
   aaataagacc ttgatgccac ttgcctcaaa aatgaataaa aatcatttca tttcggcatt 3000
   aagtgaagca tttatgagat gtatgccctt aacattaggg attgcattat tgacaattat 3060
   aggatacttt ccagttcctg cctgggtaga tttcttaaac tctattggac tggctcagca 3120
   tttttcagca gttattggtg cagttaccag tgcgctagca atttatgtaa cttataattt 3180
   tgcttattct tatgtaaatc gtcatgaata taatggccat acggccggtt tattatcaat 3240
   cgcaagtttg ttaatgctaa tgccacaaat tattactgtc cctgtagtaa aaaacattcc 3300
   aaccgaattt ccgaaatccg cggtagttga cagtgtgtca aatgttgaag catttcaaac 3360
   ggtatacacg ggtagcacag gattaattgt agcaatcata attggtttta ttgtttcatt 3420
   agtctatata caattgagca aaagaaattt agttattaaa ttaccagctg gagttcctcc 3480
   aatggttgta gattcactaa gtccagcaat tatttcaatg gtgattttct gtttgatgtt 3540
   cgggattcgt gtgggattct cttatacgcc attccatgat attttcaatt tctcaacaca 3600
   actaattcaa gcaccgttga ctggtgctgt ggcaaatcca tgggttctta tgggcatctt 3660
   tacctttggt aatttcttat ggttctttgg tatccaccct aatttaattg ggggaatttt 3720
   aaatccattg ttattaacaa tgtcatatgc taatattgat gcctatgctg ccggaaaacc 3780
   tgtaccatac ttacaaatga tgattgtgtt tgctgtgggt gcgaacgcat ggggcggaag 3840
   tggaaatact tatgggttag ttatttcaat gtttacggca aaatctgaac gctataaaca 3900
   attattaaaa ttaggtgcaa ttcctagtat tttcaatatc agtgaaccat tactttttgg 3960
   tcttccaatg atgttaaatc ctcttttctt tattcctttg gttttccaac cagcaatttt 4020
   aggaactgta gcattgggct tggcaaagat attatatatt acaaatctga atccaatgac 4080
   ggcacttctt ccttggacga caccagcacc tgtgagaatg gccatttcag gtggacttcc 4140
   atttttgatt atttttgcaa tctgtttagt cttgaatgtt cttatttact acccattctt 4200
   taaggtggcg tataataaag ctttagaaga agaaaaagca gctgttgaat tagagggttc 4260
   agaaactgcc tgatggatat tttttataaa tctggtttga acaaattata ttgacatctc 4320
   tttttctatc ctgataattc tgagaggtta ttttgggaaa tactattgaa ccatatcgag 4380
   gtggtgtggt ataatgaagg gaattaaaaa agataggaaa atttcatgga ttaagtcatc 4440
   ttacctcttt tattagtttt ttcttataat ctaatgataa catttttata attaatctat 4500
   aaaccatatc cctctttgga atcaaaattt attatctact cctttgtaga tatgttataa 4560
   tacaagtatc agatctggga gaccacaacg gtttcccact agaaataatt ttgtttaact 4620
   ttagaaagga gatatacgca tgaaaaaaaa gattatctca gctattttaa tgtctacagt 4680
   catactttct gctgcagccc cgttgtcagg tgtttacgcc tcagctggtc aaggtactca 4740
   atcagaaaac tcatgtactc actttccagg taacttgcca aacatgcttc gtgatttgcg 4800
   tgatgctttt tcacgtgtta aaactttttt tcaaatgaaa gatcaacttg ataacttgct 4860
   tttgaaagaa tcacttttgg aagattttaa aggttacctt ggttgtcaag ctttgtcaga 4920
   aatgatccaa ttttaccttg aagaagttat gccacaagct gaaaaccaag atccagatat 4980
   caaagctcac gttaactcat tgggtgaaaa ccttaaaact ttgcgtcttc gtttgcgtcg 5040
   ttgtcaccgt tttcttccat gtgaaaacaa atcaaaagct gttgaacaag ttaaaaacgc 5100
   ttttaacaaa ttgcaagaaa aaggtatcta caaagctatg tcagaatttg atatctttat 5160
   caactacatc gaagcttaca tgactatgaa aatccgtaac taactagaat taatctataa 5220
   gttactgaca aaactgtcag taactttttt tgtgggaaaa atgtattttt atgaccgtaa 5280
   agaatctgtc agtagaagtc tgaaattcgt ttaaaaatcg actagaatag gctttaacga 5340
   caagatgttt taaagagtac gctctaaatg tatttttgta tttttgtttg attacgaagt 5400
   ttaaatttaa ttgacaaatg ttttaaaatg agtataatag gacttgtaac cgattttatt 5460
   tttataaagg agaaagaaag atgaacaaac ttttacttgg aacagccttt ataggggcta 5520
   gcttactgat tggtgggggt gctcatgcag atcaaatgtt tatcgtttgt ataatcataa 5580
   tactggtgag cactctatac aactagtggg acaccaaaag aatgctaatg taagtgcggg 5640
   ttggacttat gaaggtgtcg gttggatcgc accaacaaca agttcaagcc cagtttaccg 5700
   tgtgtacaat ccaaatgcat tattacacaa aaagcaagta tgaagcccaa agtttagtaa 5760
   ataagggttg gaaatgggat aataacggaa aggcggtctt ctattctgga ggttctcaag 5820
   ccgtatatgt cgcttataat cccaatgcac aatctggcgc tcacaattac acggaaagta 5880
   gctttgagca aaatagctta ttgaatactg gttggaaata tggggcagta gcttggtacg 5940
   ggattggagt aaaaaacgaa atgttaaaca ttgctcaaat tgttagtggt aatttttcta 6000
   gtattgttgg aacttggaaa gatacttctg gaaatatgct tgaaattaat gcaatgggaa 6060
   atcttacttt aatatggaaa ggggcaaaga atcaaacctt tgaacttggc gcaggtcaac 6120
   aatttaatgg aactgcagat attgccttaa aaaatggaga gatttcccct ggtagtccac 6180
   ttaacatttt tgttgtacca acagaagttg ctttccctaa taataaaaaa gtagacgatt 6240
   caactgggca acaacgaatt tttgtgaatt attctggtac aagccctcaa atggcgaata 6300
   gtatggcagc ggtggctttt tttagagtta ttccatgatt atattaaagt tagaattgaa 6360
   taaaatgtat tattaaaaag ataatattat atcacgacaa ggcgacatct atcaacttta 6420
   ccactggtat ggaagtgacc attattacat caggaaacgc taaaacggtt gtttttacac 6480
   ccgtaaaata aataataaaa taatgtgaaa ttactgacag cattttgtca gtaatttttt 6540
   ttatcaaaat cacacaaaaa tgttcgttga cgaacaaaaa aaactatgtt ataataattc 6600
   gtatgcgaac taaaaaagaa gcgattggcc gacttttaaa agtagccagc aaccaaatgt 6660
   ctcgagaatt tgataatttt gcagctcaac ttgatttgac aggtcagcaa atgtcaattt 6720
   tagattttct tggaaatcaa agcgaagaag gttcaggaaa agaaattagt cagacgatga 6780
   ttgaattaga atttaatatc cgacgttcaa caacgacgga aattttacag cgcatggaaa 6840
   agcggctttt aattaatcga agaacaagcc tgaccgatgc ccgccaaaaa tcagttgaat 6900
   taactgaaga agggaaaaga tatttacctg aaatcagggc ttatatccaa gcacataata 6960
   aaaaagc 6967
<210> 20
   <211> 6753
   <212> DNA
   <213> Lactococcus lactis
<220>
   <221> misc_feature
   <223> Thy12
<400> 20
   attaacagcc ttttgagcag ctagctcatt attttgaaat aaatcataaa tttctttccc     60
   actatctgat ttatgattgc tagcatattt gttgtataat cgaacgagtc cattttgaac    120
   agatccatat agattgagtg aactataaaa tacatctata tcatagttga gtttgttcac    180
   aatcatgaga ccaaattctc cagcatttcg tgtagaacca cgataaagct gtttatttag    240
   caaaatggca cctccgacac ctgtacctaa agtcatgcaa ataaaatttt ggctttcttg    300
   tccattccct agccaaagtt cagctagacc tgcacaattg gcatcatttt caacataaac    360
   cggaagattt aaatgttttt gtagttctgt ccccaatgga tagccataaa gatcagttag    420
   agctcctgcc agtaataatg ttcccttttt gtcagaagtt ccgggaacac ttacaccaat    480
   tgcagatact gaatgatgag cttttaactg atgaatattt gtgagcaagc tatccataat    540
   tttttctttt tttaatgggg ttggaacttg taaatgttgt atgatcgttc catcactagt    600
   tacaagacca aattttataa atgtaccacc gatatcaatt cctattgaat aatgcatctt    660
   ttattacctc tttctctaat ttgttttagt atagcaaaat caaaaaatta attatggtat    720
   gcattataga tatgttgtat aattttcaca aaaacggaga aaactatgaa aacaatagaa    780
   cagctcatga tagattcagc agatttaatg tcagatttta ttcaattgac aatttttata    840
   ttccgcaagg aggattttca acttttttat aggagtgatg aagaagagca agctttttca     900
   aggtaatgac tccaacttat tgatagtgtt ttatgttcag ataatgcccg atgactttgt    960
   catgcagctc caccgatttt gagaacgaca gcgacttccg tcccagccgt gccaggtgct   1020
   gcctcagatt caggttatgc cgctcaattc gctgcgtata tcgcttgctg attacgtgca   1080
   gctttccctt caggcgggat tcatacagcg gccagccatc cgtcatccat atcaccacgt   1140
   caaagggtga cagcaggctc ataagacgcc ccagcgtcgc catagtgcgt tcaccgaata   1200
   cgtgcgcaac aaccgtcttc cggagactgt catacgcgta aaacagccag cgctggcgcg   1260
   atttagcccc gacatagccc cactgttcgt ccatttccgc gcagacgatg acgtcactgc   1320
   ccggctgtat gcgcgaggtt accgactgcg gcctgagttt tttaagtgac gtaaaatcgt   1380
   gttgaggcca acgcccataa tgcgggctgt tgcccggcat ccaacgccat tcatggccat   1440
   atcaatgatt ttctggtgcg taccgggttg agaagcggtg taagtgaact gcagttgcca   1500
   tgttttacgg cagtgagagc agagatagcg ctgatgtccg gcggtgcttt tgccgttacg   1560
   caccaccccg tcagtagctg aacaggaggg acagctgata gaaacagaag ccactggagc   1620
   acctcaaaaa caccatcata cactaaatca gtaagttggc agcatcaccc tttttcaaaa   1680
   gaaatcatcg ctcatttatc tcagttgccc ttgaaggaag aggtgaattt attttatatg   1740
   cctaagataa aaggatatat tacttatttt tctgtatttg gtaaagagga gtatcttcta   1800
   cttattttta aaggacaaga aaaacttgca aataatcctt tccccgttga agtaaaacaa   1860
   ttattaaaaa gtggtatttt actctatcaa atgatttttc aagaaaaatt agattatgaa 1920
   gaattatttg agaaaaatca gcatattatt tctccattgc ttgctgctaa accaattgaa 1980
   tggaatgatt ccaatacgtg aggaaagtaa attcccataa aacatatctt tttgaaaaat 2040
   atttggggga atgtgttatt cgtggagatg ttgcagagtt aaaaaaagct ttttcaaatt 2100
   atatgaataa aggaactgct ggaaaattat ctaataattc aatgcgacat aagaaaaaca 2160
   ttttgatttc agtcatcact atgactactc gttcggctat acagggagga ttacctgaag 2220
   aagaagcttt tttgatgagt gatttatata ttcaagagct tgaagaatta acggaattag 2280
   aagaaattag aacgcttgcc tataatgtga tgatcgattt tgcagataaa gtgaaacagc 2340
   atcgatattg tcaggtttct tataaaatat tatcttgtca aaagtatatt gttaatcatt 2400
   tatacgaaaa actaagtgtg agtgaaattg cagaagagct acacatgaat atttcttatt 2460
   tatcttcaca attcaaaaaa gagacagggc aaacaattac aaactttatt caggagaagc 2520
   gaatagaaga agctagagaa ttaatccttt tctcagacta tcctttttca agaatttata 2580
   ccttgttggt tttactgcca aagtcatttt ataaaaatat ttaaaaaata tactggaata 2640
   actcccaaaa agtttcaaga tcagtatatt tatcatgcct ctacatcaat atatgattga 2700
   aattaaaaaa agacctagaa tttcaaaatt gataaaatac atacctaaaa tattaattct 2760
   gtactattac gggtggagta tctactgtat aatgagggta taaattatgg aagaagggag 2820
   taaaactaaa tttattgatg gttttacgaa ttaattagga tatttttttt aaaaaccaaa 2880
   gaaaacgctt acaaacgtta aaggagtgaa tctaaagatg gacaaatttg aaaaatggct 2940
   aaataagacc ttgatgccac ttgcctcaaa aatgaataaa aatcatttca tttcggcatt 3000
   aagtgaagca tttatgagat gtatgccctt aacattaggg attgcattat tgacaattat 3060
   aggatacttt ccagttcctg cctgggtaga tttcttaaac tctattggac tggctcagca 3120
   tttttcagca gttattggtg cagttaccag tgcgctagca atttatgtaa cttataattt 3180
   tgcttattct tatgtaaatc gtcatgaata taatggccat acggccggtt tattatcaat 3240
   cgcaagtttg ttaatgctaa tgccacaaat tattactgtc cctgtagtaa aaaacattcc 3300
   aaccgaattt ccgaaatccg cggtagttga cagtgtgtca aatgttgaag catttcaaac 3360
   ggtatacacg ggtagcacag gattaattgt agcaatcata attggtttta ttgtttcatt 3420
   agtctatata caattgagca aaagaaattt agttattaaa ttaccagctg gagttcctcc 3480
   aatggttgta gattcactaa gtccagcaat tatttcaatg gtgattttct gtttgatgtt 3540
   cgggattcgt gtgggattct cttatacgcc attccatgat attttcaatt tctcaacaca 3600
   actaattcaa gcaccgttga ctggtgctgt ggcaaatcca tgggttctta tgggcatctt 3660
   tacctttggt aatttcttat ggttctttgg tatccaccct aatttaattg ggggaatttt 3720
   aaatccattg ttattaacaa tgtcatatgc taatattgat gcctatgctg ccggaaaacc 3780
   tgtaccatac ttacaaatga tgattgtgtt tgctgtgggt gcgaacgcat ggggcggaag 3840
   tggaaatact tatgggttag ttatttcaat gtttacggca aaatctgaac gctataaaca 3900
   attattaaaa ttaggtgcaa ttcctagtat tttcaatatc agtgaaccat tactttttgg 3960
   tcttccaatg atgttaaatc ctcttttctt tattcctttg gttttccaac cagcaatttt 4020
   aggaactgta gcattgggct tggcaaagat attatatatt acaaatctga atccaatgac 4080
   ggcacttctt ccttggacga caccagcacc tgtgagaatg gccatttcag gtggacttcc 4140
   atttttgatt atttttgcaa tctgtttagt cttgaatgtt cttatttact acccattctt 4200
   taaggtggcg tataataaag ctttagaaga agaaaaagca gctgttgaat tagagggttc 4260
   agaaactgcc tgatggatat tttttataaa tctggtttga acaaattata ttgacatctc 4320
   tttttctatc ctgataattc tgagaggtta ttttgggaaa tactattgaa ccatatcgag 4380
   gtggtgtggt ataatgaagg gaattaaaaa agataggaaa atttcatgaa aaaaaagatt 4440
   atctcagcta ttttaatgtc tacagtcata ctttctgctg cagccccgtt gtcaggtgtt 4500
   tacgcctcag ctggtcaagg tactcaatca gaaaactcat gtactcactt tccaggtaac 4560
   ttgccaaaca tgcttcgtga tttgcgtgat gctttttcac gtgttaaaac tttttttcaa 4620
   atgaaagatc aacttgataa cttgcttttg aaagaatcac ttttggaaga ttttaaaggt 4680
   taccttggtt gtcaagcttt gtcagaaatg atccaatttt accttgaaga agttatgcca 4740
   caagctgaaa accaagatcc agatatcaaa gctcacgtta actcattggg tgaaaacctt 4800
   aaaactttgc gtcttcgttt gcgtcgttgt caccgttttc ttccatgtga aaacaaatca 4860
   aaagctgttg aacaagttaa aaacgctttt aacaaattgc aagaaaaagg tatctacaaa 4920
   gctatgtcag aatttgatat ctttatcaac tacatcgaag cttacatgac tatgaaaatc 4980
   cgtaactaac tagaattaat ctataagtta ctgacaaaac tgtcagtaac tttttttgtg 5040
   ggaaaaatgt atttttatga ccgtaaagaa tctgtcagta gaagtctgaa attcgtttaa 5100
   aaatcgacta gaataggctt taacgacaag atgttttaaa gagtacgctc taaatgtatt 5160
   tttgtatttt tgtttgatta cgaagtttaa atttaattga caaatgtttt aaaatgagta 5220
   taataggact tgtaaccgat tttattttta taaaggagaa agaaagatga acaaactttt 5280
   acttggaaca gcctttatag ggctagcttt actgattggt gggggtgctc atgcagatca 5340
   aatgtttatc gtttgtataa tcataatact ggtgagcact ctatacaact agtgggacac 5400
   caaaagaatg ctaatgtaag tgcgggttgg acttatgaag gtgtcggttg gatcgcacca 5460
   acaacaagtt caagcccagt ttaccgtgtg tacaatccaa atgcattatt acacaaaaag 5520
   caagtatgaa gcccaaagtt tagtaaataa gggttggaaa tgggataata acggaaaggc 5580
   ggtcttctat tctggaggtt ctcaagccgt atatgtcgct tataatccca atgcacaatc 5640
   tggcgctcac aattacacgg aaagtagctt tgagcaaaat agcttattga atactggttg 5700
   gaaatatggg gcagtagctt ggtacgggat tggagtaaaa aacgaaatgt taaacattgc 5760
   tcaaattgtt agtggtaatt tttctagtat tgttggaact tggaaagata cttctggaaa 5820
   tatgcttgaa attaatgcaa tgggaaatct tactttaata tggaaagggg caaagaatca 5880
   aacctttgaa cttggcgcag gtcaacaatt taatggaact gcagatattg ccttaaaaaa 5940
   tggagagatt tcccctggta gtccacttaa catttttgtt gtaccaacag aagttgcttt 6000
   ccctaataat aaaaaagtag acgattcaac tgggcaacaa cgaatttttg tgaattattc 6060
   tggtacaagc cctcaaatgg cgaatagtat ggcagcggtg gcttttttta gagttattcc 6120
   atgattatat taaagttaga attgaataaa atgtattatt aaaaagataa tattatatca 6180
   cgacaaggcg acatctatca actttaccac tggtatggaa gtgaccatta ttacatcagg 6240
   aaacgctaaa acggttgttt ttacacccgt aaaataaata ataaaataat gtgaaattac 6300
   tgacagcatt ttgtcagtaa ttttttttat caaaatcaca caaaaatgtt cgttgacgaa 6360
   caaaaaaaac tatgttataa taattcgtat gcgaactaaa aaagaagcga ttggccgact 6420
   tttaaaagta gccagcaacc aaatgtctcg agaatttgat aattttgcag ctcaacttga 6480
   tttgacaggt cagcaaatgt caattttaga ttttcttgga aatcaaagcg aagaaggttc 6540
   aggaaaagaa attagtcaga cgatgattga attagaattt aatatccgac gttcaacaac 6600
   gacggaaatt ttacagcgca tggaaaagcg gcttttaatt aatcgaagaa caagcctgac 6660
   cgatgcccgc caaaaatcag ttgaattaac tgaagaaggg aaaagatatt tacctgaaat 6720
   cagggcttat atccaagcac ataataaaaa agc 6753
<210> 21
   <211> 6904
   <212> DNA
   <213> Lactococcus lactis
<220>
   <221> misc_feature
   <223> Thy15̅
<400> 21
   attaacagcc ttttgagcag ctagctcatt attttgaaat aaatcataaa tttctttccc     60
   actatctgat ttatgattgc tagcatattt gttgtataat cgaacgagtc cattttgaac    120
   agatccatat agattgagtg aactataaaa tacatctata tcatagttga gtttgttcac    180
   aatcatgaga ccaaattctc cagcatttcg tgtagaacca cgataaagct gtttatttag    240
   caaaatggca cctccgacac ctgtacctaa agtcatgcaa ataaaatttt ggctttcttg    300
   tccattccct agccaaagtt cagctagacc tgcacaattg gcatcatttt caacataaac    360
   cggaagattt aaatgttttt gtagttctgt ccccaatgga tagccataaa gatcagttag    420
   agctcctgcc agtaataatg ttcccttttt gtcagaagtt ccgggaacac ttacaccaat    480
   tgcagatact gaatgatgag cttttaactg atgaatattt gtgagcaagc tatccataat    540
   tttttctttt tttaatgggg ttggaacttg taaatgttgt atgatcgttc catcactagt    600
   tacaagacca aattttataa atgtaccacc gatatcaatt cctattgaat aatgcatctt    660
   ttattacctc tttctctaat ttgttttagt atagcaaaat caaaaaatta attatggtat    720
   gcattataga tatgttgtat aattttcaca aaaacggaga aaactatgaa aacaatagaa    780
   cagctcatga tagattcagc agatttaatg tcagatttta ttcaattgac aatttttata    840
   ttccgcaagg aggattttca acttttttat aggagtgatg aagaagagca agctttttca 900
   aggtaatgac tccaacttat tgatagtgtt ttatgttcag ataatgcccg atgactttgt 960
   catgcagctc caccgatttt gagaacgaca gcgacttccg tcccagccgt gccaggtgct 1020
   gcctcagatt caggttatgc cgctcaattc gctgcgtata tcgcttgctg attacgtgca 1080
   gctttccctt caggcgggat tcatacagcg gccagccatc cgtcatccat atcaccacgt 1140
   caaagggtga cagcaggctc ataagacgcc ccagcgtcgc catagtgcgt tcaccgaata 1200
   cgtgcgcaac aaccgtcttc cggagactgt catacgcgta aaacagccag cgctggcgcg 1260
   atttagcccc gacatagccc cactgttcgt ccatttccgc gcagacgatg acgtcactgc 1320
   ccggctgtat gcgcgaggtt accgactgcg gcctgagttt tttaagtgac gtaaaatcgt 1380
   gttgaggcca acgcccataa tgcgggctgt tgcccggcat ccaacgccat tcatggccat 1440
   atcaatgatt ttctggtgcg taccgggttg agaagcggtg taagtgaact gcagttgcca 1500
   tgttttacgg cagtgagagc agagatagcg ctgatgtccg gcggtgcttt tgccgttacg 1560
   caccaccccg tcagtagctg aacaggaggg acagctgata gaaacagaag ccactggagc 1620
   acctcaaaaa caccatcata cactaaatca gtaagttggc agcatcaccc tttttcaaaa 1680
   gaaatcatcg ctcatttatc tcagttgccc ttgaaggaag aggtgaattt attttatatg 1740
   cctaagataa aaggatatat tacttatttt tctgtatttg gtaaagagga gtatcttcta 1800
   cttattttta aaggacaaga aaaacttgca aataatcctt tccccgttga agtaaaacaa 1860
   ttattaaaaa gtggtatttt actctatcaa atgatttttc aagaaaaatt agattatgaa 1920
   gaattatttg agaaaaatca gcatattatt tctccattgc ttgctgctaa accaattgaa 1980
   tggaatgatt ccaatacgtg aggaaagtaa attcccataa aacatatctt tttgaaaaat 2040
   atttggggga atgtgttatt cgtggagatg ttgcagagtt aaaaaaagct ttttcaaatt 2100
   atatgaataa aggaactgct ggaaaattat ctaataattc aatgcgacat aagaaaaaca 2160
   ttttgatttc agtcatcact atgactactc gttcggctat acagggagga ttacctgaag 2220
   aagaagcttt tttgatgagt gatttatata ttcaagagct tgaagaatta acggaattag 2280
   aagaaattag aacgcttgcc tataatgtga tgatcgattt tgcagataaa gtgaaacagc 2340
   atcgatattg tcaggtttct tataaaatat tatcttgtca aaagtatatt gttaatcatt 2400
   tatacgaaaa actaagtgtg agtgaaattg cagaagagct acacatgaat atttcttatt 2460
   tatcttcaca attcaaaaaa gagacagggc aaacaattac aaactttatt caggagaagc 2520
   gaatagaaga agctagagaa ttaatccttt tctcagacta tcctttttca agaatttata 2580
   ccttgttggt tttactgcca aagtcatttt ataaaaatat ttaaaaaata tactggaata 2640
   actcccaaaa agtttcaaga tcagtatatt tatcatgcct ctacatcaat atatgattga 2700
   aattaaaaaa agacctagaa tttcaaaatt gataaaatac atacctaaaa tattaattct 2760
   gtactattac gggtggagta tctactgtat aatgagggta taaattatgg aagaagggag 2820
   taaaactaaa tttattgatg gttttacgaa ttaattagga tatttttttt aaaaaccaaa 2880
   gaaaacgctt acaaacgtta aaggagtgaa tctaaagatg gacaaatttg aaaaatggct 2940
   aaataagacc ttgatgccac ttgcctcaaa aatgaataaa aatcatttca tttcggcatt 3000
   aagtgaagca tttatgagat gtatgccctt aacattaggg attgcattat tgacaattat 3060
   aggatacttt ccagttcctg cctgggtaga tttcttaaac tctattggac tggctcagca 3120
   tttttcagca gttattggtg cagttaccag tgcgctagca atttatgtaa cttataattt 3180
   tgcttattct tatgtaaatc gtcatgaata taatggccat acggccggtt tattatcaat 3240
   cgcaagtttg ttaatgctaa tgccacaaat tattactgtc cctgtagtaa aaaacattcc 3300
   aaccgaattt ccgaaatccg cggtagttga cagtgtgtca aatgttgaag catttcaaac 3360
   ggtatacacg ggtagcacag gattaattgt agcaatcata attggtttta ttgtttcatt 3420
   agtctatata caattgagca aaagaaattt agttattaaa ttaccagctg gagttcctcc 3480
   aatggttgta gattcactaa gtccagcaat tatttcaatg gtgattttct gtttgatgtt 3540
   cgggattcgt gtgggattct cttatacgcc attccatgat attttcaatt tctcaacaca 3600
   actaattcaa gcaccgttga ctggtgctgt ggcaaatcca tgggttctta tgggcatctt 3660
   tacctttggt aatttcttat ggttctttgg tatccaccct aatttaattg gggaattttt 3720
   aaatccattg ttattaacaa tgtcatatgc taatattgat gcctatgctg ccggaaaacc 3780
   tgtaccatac ttacaaatga tgattgtgtt tgctgtgggt gcgaacgcat ggggcggaag 3840
   tggaaatact tatgggttag ttatttcaat gtttacggca aaatctgaac gctataaaca 3900
   attattaaaa ttaggtgcaa ttcctagtat tttcaatatc agtgaaccat tactttttgg 3960
   tcttccaatg atgttaaatc ctcttttctt tattcctttg gttttccaac cagcaatttt 4020
   aggaactgta gcattgggct tggcaaagat attatatatt acaaatctga atccaatgac 4080
   ggcacttctt ccttggacga caccagcacc tgtgagaatg gccatttcag gtggacttcc 4140
   atttttgatt atttttgcaa tctgtttagt cttgaatgtt cttatttact acccattctt 4200
   taaggtggcg tataataaag ctttagaaga agaaaaagca gctgttgaat tagagggttc 4260
   agaaactgcc tgatggatat tttttataaa tctggtttga acaaattata ttgacatctc 4320
   tttttctatc ctgataattc tgagaggtta ttttgggaaa tactagatta agtcatctta 4380
   cctcttttat tagttttttc ttataatcta atgataacat ttttataatt aatctataaa 4440
   ccatatccct ctttggaatc aaaatttatt atctactcct ttgtagatat gttataatac 4500
   aagtatcaga tctgggagac cacaacggtt tcccactaga aataattttg tttaacttta 4560
   gaaaggagat atacgcatga aaaaaaagat tatctcagct attttaatgt ctacagtcat 4620
   actttctgct gcagccccgt tgtcaggtgt ttacgcctca gctggtcaag gtactcaatc 4680
   agaaaactca tgtactcact ttccaggtaa cttgccaaac atgcttcgtg atttgcgtga 4740
   tgctttttca cgtgttaaaa ctttttttca aatgaaagat caacttgata acttgctttt 4800
   gaaagaatca cttttggaag attttaaagg ttaccttggt tgtcaagctt tgtcagaaat 4860
   gatccaattt taccttgaag aagttatgcc acaagctgaa aaccaagatc cagatatcaa 4920
   agctcacgtt aactcattgg gtgaaaacct taaaactttg cgtcttcgtt tgcgtcgttg 4980
   tcaccgtttt cttccatgtg aaaacaaatc aaaagctgtt gaacaagtta aaaacgcttt 5040
   taacaaattg caagaaaaag gtatctacaa agctatgtca gaatttgata tctttatcaa 5100
   ctacatcgaa gcttacatga ctatgaaaat ccgtaactaa ctagaattaa tctataagtt 5160
   actgacaaaa ctgtcagtaa ctttttttgt gggaaaaatg tatttttatg accgtaaaga 5220
   atctgtcagt agaagtctga aattcgttta aaaatcgact agaataggct ttaacgacaa 5280
   gatgttttaa agagtacgct ctaaatgtat ttttgtattt ttgtttgatt acgaagttta 5340
   aatttaattg acaaatgttt taaaatgagt ataataggac ttgtaaccga ttttattttt 5400
   ataaaggaga aagaaagatg aacaaacttt tacttggaac agcctttata ggggctagct 5460
   tactgattgg tgggggtgct catgcagatc aaatgtttat cgtttgtata atcataatac 5520
   tggtgagcac tctatacaac tagtgggaca ccaaaagaat gctaatgtaa gtgcgggttg 5580
   gacttatgaa ggtgtcggtt ggatcgcacc aacaacaagt tcaagcccag tttaccgtgt 5640
   gtacaatcca aatgcattat tacacaaaaa gcaagtatga agcccaaagt ttagtaaata 5700
   agggttggaa atgggataat aacggaaagg cggtcttcta ttctggaggt tctcaagccg 5760
   tatatgtcgc ttataatccc aatgcacaat ctggcgctca caattacacg gaaagtagct 5820
   ttgagcaaaa tagcttattg aatactggtt ggaaatatgg ggcagtagct tggtacggga 5880
   ttggagtaaa aaacgaaatg ttaaacattg ctcaaattgt tagtggtaat ttttctagta 5940
   ttgttggaac ttggaaagat acttctggaa atatgcttga aattaatgca atgggaaatc 6000
   ttactttaat atggaaaggg gcaaagaatc aaacctttga acttggcgca ggtcaacaat 6060
   ttaatggaac tgcagatatt gccttaaaaa atggagagat ttcccctggt agtccactta 6120
   acatttttgt tgtaccaaca gaagttgctt tccctaataa taaaaaagta gacgattcaa 6180
   ctgggcaaca acgaattttt gtgaattatt ctggtacaag ccctcaaatg gcgaatagta 6240
   tggcagcggt ggcttttttt agagttattc catgattata ttaaagttag aattgaataa 6300
   aatgtattat taaaaagata atattatatc acgacaaggc gacatctatc aactttacca 6360
   ctggtatgga agtgaccatt attacatcag gaaacgctaa aacggttgtt tttacacccg 6420
   taaaataaat aataaaataa tgtgaaatta ctgacagcat tttgtcagta atttttttta 6480
   tcaaaatcac acaaaaatgt tcgttgacga acaaaaaaaa ctatgttata ataattcgta 6540
   tgcgaactaa aaaagaagcg attggccgac ttttaaaagt agccagcaac caaatgtctc 6600
   gagaatttga taattttgca gctcaacttg atttgacagg tcagcaaatg tcaattttag 6660
   attttcttgg aaatcaaagc gaagaaggtt caggaaaaga aattagtcag acgatgattg 6720
   aattagaatt taatatccga cgttcaacaa cgacggaaat tttacagcgc atggaaaagc 6780
   ggcttttaat taatcgaaga acaagcctga ccgatgcccg ccaaaaatca gttgaattaa 6840
   ctgaagaagg gaaaagatat ttacctgaaa tcagggctta tatccaagca cataataaaa 6900
   aagc 6904
<210> 22
   <211> 6964
   <212> DNA
   <213> Lactococcus lactis
<220>
   <221> misc_feature
   <223> Thy16
<400> 22
   attaacagcc ttttgagcag ctagctcatt attttgaaat aaatcataaa tttctttccc     60
   actatctgat ttatgattgc tagcatattt gttgtataat cgaacgagtc cattttgaac    120
   agatccatat agattgagtg aactataaaa tacatctata tcatagttga gtttgttcac    180
   aatcatgaga ccaaattctc cagcatttcg tgtagaacca cgataaagct gtttatttag    240
   caaaatggca cctccgacac ctgtacctaa agtcatgcaa ataaaatttt ggctttcttg    300
   tccattccct agccaaagtt cagctagacc tgcacaattg gcatcatttt caacataaac    360
   cggaagattt aaatgttttt gtagttctgt ccccaatgga tagccataaa gatcagttag    420
   agctcctgcc agtaataatg ttcccttttt gtcagaagtt ccgggaacac ttacaccaat    480
   tgcagatact gaatgatgag cttttaactg atgaatattt gtgagcaagc tatccataat    540
   tttttctttt tttaatgggg ttggaacttg taaatgttgt atgatcgttc catcactagt    600
   tacaagacca aattttataa atgtaccacc gatatcaatt cctattgaat aatgcatctt    660
   ttattacctc tttctctaat ttgttttagt atagcaaaat caaaaaatta attatggtat    720
   gcattataga tatgttgtat aattttcaca aaaacggaga aaactatgaa aacaatagaa    780
   cagctcatga tagattcagc agatttaatg tcagatttta ttcaattgac aatttttata    840
   ttccgcaagg aggattttca acttttttat aggagtgatg aagaagagca agctttttca    900
   aggtaatgac tccaacttat tgatagtgtt ttatgttcag ataatgcccg atgactttgt    960
   catgcagctc caccgatttt gagaacgaca gcgacttccg tcccagccgt gccaggtgct   1020
   gcctcagatt caggttatgc cgctcaattc gctgcgtata tcgcttgctg attacgtgca   1080
   gctttccctt caggcgggat tcatacagcg gccagccatc cgtcatccat atcaccacgt   1140
   caaagggtga cagcaggctc ataagacgcc ccagcgtcgc catagtgcgt tcaccgaata   1200
   cgtgcgcaac aaccgtcttc cggagactgt catacgcgta aaacagccag cgctggcgcg   1260
   atttagcccc gacatagccc cactgttcgt ccatttccgc gcagacgatg acgtcactgc   1320
   ccggctgtat gcgcgaggtt accgactgcg gcctgagttt tttaagtgac gtaaaatcgt   1380
   gttgaggcca acgcccataa tgcgggctgt tgcccggcat ccaacgccat tcatggccat   1440
   atcaatgatt ttctggtgcg taccgggttg agaagcggtg taagtgaact gcagttgcca   1500
   tgttttacgg cagtgagagc agagatagcg ctgatgtccg gcggtgcttt tgccgttacg   1560
   caccaccccg tcagtagctg aacaggaggg acagctgata gaaacagaag ccactggagc   1620
   acctcaaaaa caccatcata cactaaatca gtaagttggc agcatcaccc tttttcaaaa   1680
   gaaatcatcg ctcatttatc tcagttgccc ttgaaggaag aggtgaattt attttatatg 1740
   cctaagataa aaggatatat tacttatttt tctgtatttg gtaaagagga gtatcttcta 1800
   cttattttta aaggacaaga aaaacttgca aataatcctt tccccgttga agtaaaacaa 1860
   ttattaaaaa gtggtatttt actctatcaa atgatttttc aagaaaaatt agattatgaa 1920
   gaattatttg agaaaaatca gcatattatt tctccattgc ttgctgctaa accaattgaa 1980
   tggaatgatt ccaatacgtg aggaaagtaa attcccataa aacatatctt tttgaaaaat 2040
   atttggggga atgtgttatt cgtggagatg ttgcagagtt aaaaaaagct ttttcaaatt 2100
   atatgaataa aggaactgct ggaaaattat ctaataattc aatgcgacat aagaaaaaca 2160
   ttttgatttc agtcatcact atgactactc gttcggctat acagggagga ttacctgaag 2220
   aagaagcttt tttgatgagt gatttatata ttcaagagct tgaagaatta acggaattag 2280
   aagaaattag aacgcttgcc tataatgtga tgatcgattt tgcagataaa gtgaaacagc 2340
   atcgatattg tcaggtttct tataaaatat tatcttgtca aaagtatatt gttaatcatt 2400
   tatacgaaaa actaagtgtg agtgaaattg cagaagagct acacatgaat atttcttatt 2460
   tatcttcaca attcaaaaaa gagacagggc aaacaattac aaactttatt caggagaagc 2520
   gaatagaaga agctagagaa ttaatccttt tctcagacta tcctttttca agaatttata 2580
   ccttgttggt tttactgcca aagtcatttt ataaaaatat ttaaaaaata tactggaata 2640
   actcccaaaa agtttcaaga tcagtatatt tatcatgcct ctacatcaat atatgattga 2700
   aattaaaaaa agacctagaa tttcaaaatt gataaaatac atacctaaaa tattaattct 2760
   gtactattac gggtggagta tctactgtat aatgagggta taaattatgg aagaagggag 2820
   taaaactaaa tttattgatg gttttacgaa ttaattagga tatttttttt aaaaaccaaa 2880
   gaaaacgctt acaaacgtta aaggagtgaa tctaaagatg gacaaatttg aaaaatggct 2940
   aaataagacc ttgatgccac ttgcctcaaa aatgaataaa aatcatttca tttcggcatt 3000
   aagtgaagca tttatgagat gtatgccctt aacattaggg attgcattat tgacaattat 3060
   aggatacttt ccagttcctg cctgggtaga tttcttaaac tctattggac tggctcagca 3120
   tttttcagca gttattggtg cagttaccag tgcgctagca atttatgtaa cttataattt 3180
   tgcttattct tatgtaaatc gtcatgaata taatggccat acggccggtt tattatcaat 3240
   cgcaagtttg ttaatgctaa tgccacaaat tattactgtc cctgtagtaa aaaacattcc 3300
   aaccgaattt ccgaaatccg cggtagttga cagtgtgtca aatgttgaag catttcaaac 3360
   ggtatacacg ggtagcacag gattaattgt agcaatcata attggtttta ttgtttcatt 3420
   agtctatata caattgagca aaagaaattt agttattaaa ttaccagctg gagttcctcc 3480
   aatggttgta gattcactaa gtccagcaat tatttcaatg gtgattttct gtttgatgtt 3540
   cgggattcgt gtgggattct cttatacgcc attccatgat attttcaatt tctcaacaca 3600
   actaattcaa gcaccgttga ctggtgctgt ggcaaatcca tgggttctta tgggcatctt 3660
   tacctttggt aatttcttat ggttctttgg tatccaccct aatttaattg gggaattttt 3720
   aaatccattg ttattaacaa tgtcatatgc taatattgat gcctatgctg ccggaaaacc 3780
   tgtaccatac ttacaaatga tgattgtgtt tgctgtgggt gcgaacgcat ggggcggaag 3840
   tggaaatact tatgggttag ttatttcaat gtttacggca aaatctgaac gctataaaca 3900
   attattaaaa ttaggtgcaa ttcctagtat tttcaatatc agtgaaccat tactttttgg 3960
   tcttccaatg atgttaaatc ctcttttctt tattcctttg gttttccaac cagcaatttt 4020
   aggaactgta gcattgggct tggcaaagat attatatatt acaaatctga atccaatgac 4080
   ggcacttctt ccttggacga caccagcacc tgtgagaatg gccatttcag gtggacttcc 4140
   atttttgatt atttttgcaa tctgtttagt cttgaatgtt cttatttact acccattctt 4200
   taaggtggcg tataataaag ctttagaaga agaaaaagca gctgttgaat tagagggttc 4260
   agaaactgcc tgatggatat tttttataaa tctggtttga acaaattata ttgacatctc 4320
   tttttctatc ctgataattc tgagaggtta ttttgggaaa tactattgaa ccatatcgag 4380
   gtggtgtggt ataatgaagg gaattaaaaa agataggaaa atttcgatta agtcatctta 4440
   cctcttttat tagttttttc ttataatcta atgataacat ttttataatt aatctataaa 4500
   ccatatccct ctttggaatc aaaatttatt atctactcct ttgtagatat gttataatac 4560
   aagtatcaga tctgggagac cacaacggtt tcccactaga aataattttg tttaacttta 4620
   gaaaggagat atacgcatga aaaaaaagat tatctcagct attttaatgt ctacagtcat 4680
   actttctgct gcagccccgt tgtcaggtgt ttacgcctca gctggtcaag gtactcaatc 4740
   agaaaactca tgtactcact ttccaggtaa cttgccaaac atgcttcgtg atttgcgtga 4800
   tgctttttca cgtgttaaaa ctttttttca aatgaaagat caacttgata acttgctttt 4860
   gaaagaatca cttttggaag attttaaagg ttaccttggt tgtcaagctt tgtcagaaat 4920
   gatccaattt taccttgaag aagttatgcc acaagctgaa aaccaagatc cagatatcaa 4980
   agctcacgtt aactcattgg gtgaaaacct taaaactttg cgtcttcgtt tgcgtcgttg 5040
   tcaccgtttt cttccatgtg aaaacaaatc aaaagctgtt gaacaagtta aaaacgcttt 5100
   taacaaattg caagaaaaag gtatctacaa agctatgtca gaatttgata tctttatcaa 5160
   ctacatcgaa gcttacatga ctatgaaaat ccgtaactaa ctagaattaa tctataagtt 5220
   actgacaaaa ctgtcagtaa ctttttttgt gggaaaaatg tatttttatg accgtaaaga 5280
   atctgtcagt agaagtctga aattcgttta aaaatcgact agaataggct ttaacgacaa 5340
   gatgttttaa agagtacgct ctaaatgtat ttttgtattt ttgtttgatt acgaagttta 5400
   aatttaattg acaaatgttt taaaatgagt ataataggac ttgtaaccga ttttattttt 5460
   ataaaggaga aagaaagatg aacaaacttt tacttggaac agcctttata ggggctagct 5520
   tactgattgg tgggggtgct catgcagatc aaatgtttat cgtttgtata atcataatac 5580
   tggtgagcac tctatacaac tagtgggaca ccaaaagaat gctaatgtaa gtgcgggttg 5640
   gacttatgaa ggtgtcggtt ggatcgcacc aacaacaagt tcaagcccag tttaccgtgt 5700
   gtacaatcca aatgcattat tacacaaaaa gcaagtatga agcccaaagt ttagtaaata 5760
   agggttggaa atgggataat aacggaaagg cggtcttcta ttctggaggt tctcaagccg   5820
   tatatgtcgc ttataatccc aatgcacaat ctggcgctca caattacacg gaaagtagct   5880
   ttgagcaaaa tagcttattg aatactggtt ggaaatatgg ggcagtagct tggtacggga   5940
   ttggagtaaa aaacgaaatg ttaaacattg ctcaaattgt tagtggtaat ttttctagta   6000
   ttgttggaac ttggaaagat acttctggaa atatgcttga aattaatgca atgggaaatc   6060
   ttactttaat atggaaaggg gcaaagaatc aaacctttga acttggcgca ggtcaacaat   6120
   ttaatggaac tgcagatatt gccttaaaaa atggagagat ttcccctggt agtccactta   6180
   acatttttgt tgtaccaaca gaagttgctt tccctaataa taaaaaagta gacgattcaa   6240
   ctgggcaaca acgaattttt gtgaattatt ctggtacaag ccctcaaatg gcgaatagta   6300
   tggcagcggt ggcttttttt agagttattc catgattata ttaaagttag aattgaataa   6360
   aatgtattat taaaaagata atattatatc acgacaaggc gacatctatc aactttacca   6420
   ctggtatgga agtgaccatt attacatcag gaaacgctaa aacggttgtt tttacacccg   6480
   taaaataaat aataaaataa tgtgaaatta ctgacagcat tttgtcagta atttttttta   6540
   tcaaaatcac acaaaaatgt tcgttgacga acaaaaaaaa ctatgttata ataattcgta   6600
   tgcgaactaa aaaagaagcg attggccgac ttttaaaagt agccagcaac caaatgtctc   6660
   gagaatttga taattttgca gctcaacttg atttgacagg tcagcaaatg tcaattttag   6720
   attttcttgg aaatcaaagc gaagaaggtt caggaaaaga aattagtcag acgatgattg   6780
   aattagaatt taatatccga cgttcaacaa cgacggaaat tttacagcgc atggaaaagc   6840
   ggcttttaat taatcgaaga acaagcctga ccgatgcccg ccaaaaatca gttgaattaa   6900
   ctgaagaagg gaaaagatat ttacctgaaa tcagggctta tatccaagca cataataaaa   6960
   aagc                                                                6964
<210> 23
   <211> 4998
   <212> DNA
   <213> Artificial Sequence
<220>
   <223> pOThy11
<400> 23
   aatttcatgg attaagtcat cttacctctt ttattagttt tttcttataa tctaatgata     60
   acatttttat aattaatcta taaaccatat ccctctttgg aatcaaaatt tattatctac    120
   tcctttgtag atatgttata atacaagtat cagatctggg agaccacaac ggtttcccac    180
   tagaaataat tttgtttaac tttagaaagg agatatacgc atgaaaaaaa agattatctc    240
   agctatttta atgtctacag tcatactttc tgctgcagcc ccgttgtcag gtgtttacgc    300
   ctcagctggt caaggtactc aatcagaaaa ctcatgtact cactttccag gtaacttgcc    360
   aaacatgctt cgtgatttgc gtgatgcttt ttcacgtgtt aaaacttttt ttcaaatgaa    420
   agatcaactt gataacttgc ttttgaaaga atcacttttg gaagatttta aaggttacct    480
   tggttgtcaa gctttgtcag aaatgatcca attttacctt gaagaagtta tgccacaagc 540
   tgaaaaccaa gatccagata tcaaagctca cgttaactca ttgggtgaaa accttaaaac 600
   tttgcgtctt cgtttgcgtc gttgtcaccg ttttcttcca tgtgaaaaca aatcaaaagc 660
   tgttgaacaa gttaaaaacg cttttaacaa attgcaagaa aaaggtatct acaaagctat 720
   gtcagaattt gatatcttta tcaactacat cgaagcttac atgactatga aaatccgtaa 780
   ctaactagaa ttaatctata agttactgac aaaactgtca gtaacttttt ttgtgggaaa 840
   aatgtatttt tatgaccgta aagaatctgt cagtagaagt ctgaaattcg tttaaaaatc 900
   gactagaata ggctttaacg acaagatgtt ttaaagagta cgctctaaat gtatttttgt 960
   atttttgttt gattacgaag tttaaattta attgacaaat gttttaaaat gagtataata 1020
   ggacttgtaa ccgattttat ttttataaag gagaaagaaa gatgaacaaa cttttacttg 1080
   gaacagcctt tataggggct agcttactga ttggtggggg tgctcatgca gatcaaatgt 1140
   ttatcgtttg tataatcata atactggtga gcactctata caactagtgg gacaccaaaa 1200
   gaatgctaat gtaagtgcgg gttggactta tgaaggtgtc ggttggatcg caccaacaac 1260
   aagttcaagc ccagtttacc gtgtgtacaa tccaaatgca ttattacaca aaaagcaagt 1320
   atgaagccca aagtttagta aataagggtt ggaaatggga taataacgga aaggcggtct 1380
   tctattctgg aggttctcaa gccgtatatg tcgcttataa tcccaatgca caatctggcg 1440
   ctcacaatta cacggaaagt agctttgagc aaaatagctt attgaatact ggttggaaat 1500
   atggggcagt agcttggtac gggattggag taaaaaacga aatgttaaac attgctcaaa 1560
   ttgttagtgg taatttttct agtattgttg gaacttggaa agatacttct ggaaatatgc 1620
   ttgaaattaa tgcaatggga aatcttactt taatatggaa aggggcaaag aatcaaacct 1680
   ttgaacttgg cgcaggtcaa caatttaatg gaactgcaga tattgcctta aaaaatggag 1740
   agatttcccc tggtagtcca cttaacattt ttgttgtacc aacagaagtg aattcactgg 1800
   ccgtcgtttt acaacgtcgt gactgggaaa accctggcgt tacccaactt aatcgccttg 1860
   cagcacatcc ccctttcgcc agctggcgta atagcgaaga ggcccgcacc gatcgccctt 1920
   cccaacagtt gcgcagcctg aatggcgaat ggcgcctgat gcggtatttt ctccttacgc 1980
   atctgtgcgg tatttcacac cgcatatggt gcactagaac tagcgattct gaaatcacca 2040
   tttaaaaaac tccaatcaaa taattttata aagttagtgt atcactttgt aatcataaaa 2100
   acaacaataa agctacttaa atatagattt ataaaaaacg ttggcgaaaa cgttggcgat 2160
   tcgttggcga ttgaaaaacc ccttaaaccc ttgagccagt tgggatagag cgtttttggc 2220
   acaaaaattg gcactcggca cttaatgggg ggtcgtagta cggaagcaaa attcgcttcc 2280
   tttcccccca tttttttcca aattccaaat ttttttcaaa aattttccag cgctaccgct 2340
   cggcaaaatt gcaagcaatt tttaaaatca aacccatgag ggaatttcat tccctcatac 2400
   tcccttgagc ctcctccaac cgaaatagaa gggcgctgcg cttattattt cattcagtca 2460
   tcggctttca taatctaaca gacaacatct tcgctgcaaa gccacgctac gctcaagggc 2520
   ttttacgcta cgataacgcc tgttttaacg attatgccga taactaaacg aaataaacgc 2580
   taaaacgtct cagaaacgat tttgagacgt tttaataaaa aatcgctagt ccgaggcctc 2640
   gacccgattc acaaaaaata ggcacacgaa aaacaagtta agggatgcag tttatgcatc 2700
   ccttaactta cttattaaat aatttatagc tattgaaaag agataagaat tgttcaaagc 2760
   taatattgtt taaatcgtca attcctgcat gttttaagga attgttaaat tgattttttg 2820
   taaatatttt cttgtattct ttgttaaccc atttcataac gaaataatta tacttttgtt 2880
   tatctttgtg tgatattctt gatttttttc tacttaatct gataagtgag ctattcactt 2940
   taggtttagg atgaaaatat tctcttggaa ccatacttaa tatagaaata tcaacttctg 3000
   ccattaaaag taatgccaat gagcgttttg tatttaataa tcttttagca aacccgtatt 3060
   ccacgattaa ataaatctca ttagctatac tatcaaaaac aattttgcgt attatatccg 3120
   tacttatgtt ataaggtata ttaccatata ttttatagga ttggttttta ggaaatttaa 3180
   actgcaatat atccttgttt aaaacttgga aattatcgtg atcaacaagt ttattttctg 3240
   tagttttgca taatttatgg tctatttcaa tggcagttac gaaattacac ctctttacta 3300
   attcaagggt aaaatggcct tttcctgagc cgatttcaaa gatattatca tgttcattta 3360
   atcttatatt tgtcattatt ttatctatat tatgttttga agtaataaag ttttgactgt 3420
   gttttatatt tttctcgttc attataaccc tctttaattt ggttatatga attttgctta 3480
   ttaacgattc attataacca cttatttttt gtttggttga taatgaactg tgctgattac 3540
   aaaaatacta aaaatgccca tattttttcc tccttataaa attagtataa ttatagcacg 3600
   ggtcgagatc catgttcttt cctgcgttat cccctgattc tgtggataac cgtattaccg 3660
   cctttgagtg agctgatacc gctcgccgca gccgaacgac cgagcgcagc gagtcagtga 3720
   gcgaggaagc ggaagagcgc ccaatacgca aaccgcctct ccccgcgcgt tggccgattc 3780
   attaatgcag ctggcacgac aggtttcccg actggaaagc gggcagtgag cgcaacgcaa 3840
   ttaatgtgag ttagctcact cattaggcac cccaggcttt acactttatg cttccggctc 3900
   gtatgttgtg tggaattgtg agcggataac aatttcacac aggaaacagc tatgaccatg 3960
   attacgccaa gcttgcatgc ctgcaggtcg actctagagg atcctatata caattgagca 4020
   aaagaaattt agttattaaa ttaccagctg gagttcctcc aatggttgta gattcactaa 4080
   gtccagcaat tatttcaatg gtgattttct gtttgatgtt cgggattcgt gtgggattct 4140
   cttatacgcc attccatgat attttcaatt tctcaacaca actaattcaa gcaccgttga 4200
   ctggtgctgt ggcaaatcca tgggttctta tgggcatctt tacctttggt aatttcttat 4260
   ggttctttgg tatccaccct aatttaattg ggggaatttt aaatccattg ttattaacaa 4320
   tgtcatatgc taatattgat gcctatgctg ccggaaaacc tgtaccatac ttacaaatga 4380
   tgattgtgtt tgctgtgggt gcgaacgcat ggggcggaag tggaaatact tatgggttag 4440
   ttatttcaat gtttacggca aaatctgaac gctataaaca attattaaaa ttaggtgcaa 4500
   ttcctagtat tttcaatatc agtgaaccat tactttttgg tcttccaatg atgttaaatc 4560
   ctcttttctt tattcctttg gttttccaac cagcaatttt aggaactgta gcattgggct 4620
   tggcaaagat attatatatt acaaatctga atccaatgac ggcacttctt ccttggacga 4680
   caccagcacc tgtgagaatg gccatttcag gtggacttcc atttttgatt atttttgcaa 4740
   tctgtttagt cttgaatgtt cttatttact acccattctt taaggtggcg tataataaag 4800
   ctttagaaga agaaaaagca gctgttgaat tagagggttc agaaactgcc tgatggatat 4860
   tttttataaa tctggtttga acaaattata ttgacatctc tttttctatc ctgataattc 4920
   tgagaggtta ttttgggaaa tactattgaa ccatatcgag gtgtgtggta taatgaaggg 4980
   aattaaaaaa gataggaa 4998
<210> 24
   <211> 4784
   <212> DNA
   <213> Artificial Sequence
<220>
   <223> pOThy12
<400> 24
   gggttcagaa actgcctgat ggatattttt tataaatctg gtttgaacaa attatattga     60
   catctctttt tctatcctga taattctgag aggttatttt gggaaatact attgaaccat    120
   atcgaggtgt gtggtataat gaagggaatt aaaaaagata ggaaaatttc atgaaaaaaa    180
   agattatctc agctatttta atgtctacag tcatactttc tgctgcagcc ccgttgtcag    240
   gtgtttacgc ctcagctggt caaggtactc aatcagaaaa ctcatgtact cactttccag    300
   gtaacttgcc aaacatgctt cgtgatttgc gtgatgcttt ttcacgtgtt aaaacttttt    360
   ttcaaatgaa agatcaactt gataacttgc ttttgaaaga atcacttttg gaagatttta    420
   aaggttacct tggttgtcaa gctttgtcag aaatgatcca attttacctt gaagaagtta    480
   tgccacaagc tgaaaaccaa gatccagata tcaaagctca cgttaactca ttgggtgaaa    540
   accttaaaac tttgcgtctt cgtttgcgtc gttgtcaccg ttttcttcca tgtgaaaaca    600
   aatcaaaagc tgttgaacaa gttaaaaacg cttttaacaa attgcaagaa aaaggtatct    660
   acaaagctat gtcagaattt gatatcttta tcaactacat cgaagcttac atgactatga    720
   aaatccgtaa ctaactagaa ttaatctata agttactgac aaaactgtca gtaacttttt    780
   ttgtgggaaa aatgtatttt tatgaccgta aagaatctgt cagtagaagt ctgaaattcg    840
   tttaaaaatc gactagaata ggctttaacg acaagatgtt ttaaagagta cgctctaaat    900
   gtatttttgt atttttgttt gattacgaag tttaaattta attgacaaat gttttaaaat    960
   gagtataata ggacttgtaa ccgattttat ttttataaag gagaaagaaa gatgaacaaa   1020
   cttttacttg gaacagcctt tataggggct agcttactga ttggtggggg tgctcatgca  1080
   gatcaaatgt ttatcgtttg tataatcata atactggtga gcactctata caactagtgg   1140
   gacaccaaaa gaatgctaat gtaagtgcgg gttggactta tgaaggtgtc ggttggatcg   1200
   caccaacaac aagttcaagc ccagtttacc gtgtgtacaa tccaaatgca ttattacaca   1260
   aaaagcaagt atgaagccca aagtttagta aataagggtt ggaaatggga taataacgga 1320
   aaggcggtct tctattctgg aggttctcaa gccgtatatg tcgcttataa tcccaatgca 1380
   caatctggcg ctcacaatta cacggaaagt agctttgagc aaaatagctt attgaatact 1440
   ggttggaaat atggggcagt agcttggtac gggattggag taaaaaacga aatgttaaac 1500
   attgctcaaa ttgttagtgg taatttttct agtattgttg gaacttggaa agatacttct 1560
   ggaaatatgc ttgaaattaa tgcaatggga aatcttactt taatatggaa aggggcaaag 1620
   aatcaaacct ttgaacttgg cgcaggtcaa caatttaatg gaactgcaga tattgcctta 1680
   aaaaatggag agatttcccc tggtagtcca cttaacattt ttgttgtacc aacagaagtg 1740
   aattcactgg ccgtcgtttt acaacgtcgt gactgggaaa accctggcgt tacccaactt 1800
   aatcgccttg cagcacatcc ccctttcgcc agctggcgta atagcgaaga ggcccgcacc 1860
   gatcgccctt cccaacagtt gcgcagcctg aatggcgaat ggcgcctgat gcggtatttt 1920
   ctccttacgc atctgtgcgg tatttcacac cgcatatggt gcactagaac tagcgattct 1980
   gaaatcacca tttaaaaaac tccaatcaaa taattttata aagttagtgt atcactttgt 2040
   aatcataaaa acaacaataa agctacttaa atatagattt ataaaaaacg ttggcgaaaa 2100
   cgttggcgat tcgttggcga ttgaaaaacc ccttaaaccc ttgagccagt tgggatagag 2160
   cgtttttggc acaaaaattg gcactcggca cttaatgggg ggtcgtagta cggaagcaaa 2220
   attcgcttcc tttcccccca tttttttcca aattccaaat ttttttcaaa aattttccag 2280
   cgctaccgct cggcaaaatt gcaagcaatt tttaaaatca aacccatgag ggaatttcat 2340
   tccctcatac tcccttgagc ctcctccaac cgaaatagaa gggcgctgcg cttattattt 2400
   cattcagtca tcggctttca taatctaaca gacaacatct tcgctgcaaa gccacgctac 2460
   gctcaagggc ttttacgcta cgataacgcc tgttttaacg attatgccga taactaaacg 2520
   aaataaacgc taaaacgtct cagaaacgat tttgagacgt tttaataaaa aatcgctagt 2580
   ccgaggcctc gacccgattc acaaaaaata ggcacacgaa aaacaagtta agggatgcag 2640
   tttatgcatc ccttaactta cttattaaat aatttatagc tattgaaaag agataagaat 2700
   tgttcaaagc taatattgtt taaatcgtca attcctgcat gttttaagga attgttaaat 2760
   tgattttttg taaatatttt cttgtattct ttgttaaccc atttcataac gaaataatta 2820
   tacttttgtt tatctttgtg tgatattctt gatttttttc tacttaatct gataagtgag 2880
   ctattcactt taggtttagg atgaaaatat tctcttggaa ccatacttaa tatagaaata 2940
   tcaacttctg ccattaaaag taatgccaat gagcgttttg tatttaataa tcttttagca 3000
   aacccgtatt ccacgattaa ataaatctca ttagctatac tatcaaaaac aattttgcgt 3060
   attatatccg tacttatgtt ataaggtata ttaccatata ttttatagga ttggttttta 3120
   ggaaatttaa actgcaatat atccttgttt aaaacttgga aattatcgtg atcaacaagt 3180
   ttattttctg tagttttgca taatttatgg tctatttcaa tggcagttac gaaattacac 3240
   ctctttacta attcaagggt aaaatggcct tttcctgagc cgatttcaaa gatattatca 3300
   tgttcattta atcttatatt tgtcattatt ttatctatat tatgttttga agtaataaag   3360
   ttttgactgt gttttatatt tttctcgttc attataaccc tctttaattt ggttatatga   3420
   attttgctta ttaacgattc attataacca cttatttttt gtttggttga taatgaactg   3480
   tgctgattac aaaaatacta aaaatgccca tattttttcc tccttataaa attagtataa   3540
   ttatagcacg ggtcgagatc catgttcttt cctgcgttat cccctgattc tgtggataac   3600
   cgtattaccg cctttgagtg agctgatacc gctcgccgca gccgaacgac cgagcgcagc   3660
   gagtcagtga gcgaggaagc ggaagagcgc ccaatacgca aaccgcctct ccccgcgcgt   3720
   tggccgattc attaatgcag ctggcacgac aggtttcccg actggaaagc gggcagtgag   3780
   cgcaacgcaa ttaatgtgag ttagctcact cattaggcac cccaggcttt acactttatg   3840
   cttccggctc gtatgttgtg tggaattgtg agcggataac aatttcacac aggaaacagc   3900
   tatgaccatg attacgccaa gcttgcatgc ctgcaggtcg actctagagg atcctatata   3960
   caattgagca aaagaaattt agttattaaa ttaccagctg gagttcctcc aatggttgta   4020
   gattcactaa gtccagcaat tatttcaatg gtgattttct gtttgatgtt cgggattcgt   4080
   gtgggattct cttatacgcc attccatgat attttcaatt tctcaacaca actaattcaa   4140
   gcaccgttga ctggtgctgt ggcaaatcca tgggttctta tgggcatctt tacctttggt   4200
   aatttcttat ggttctttgg tatccaccct aatttaattg ggggaatttt aaatccattg   4260
   ttattaacaa tgtcatatgc taatattgat gcctatgctg ccggaaaacc tgtaccatac   4320
   ttacaaatga tgattgtgtt tgctgtgggt gcgaacgcat ggggcggaag tggaaatact   4380
   tatgggttag ttatttcaat gtttacggca aaatctgaac gctataaaca attattaaaa   4440
   ttaggtgcaa ttcctagtat tttcaatatc agtgaaccat tactttttgg tcttccaatg   4500
   atgttaaatc ctcttttctt tattcctttg gttttccaac cagcaatttt aggaactgta   4560
   gcattgggct tggcaaagat attatatatt acaaatctga atccaatgac ggcacttctt   4620
   ccttggacga caccagcacc tgtgagaatg gccatttcag gtggacttcc atttttgatt   4680
   atttttgcaa tctgtttagt cttgaatgtt cttatttact acccattctt taaggtggcg   4740
   tataataaag ctttagaaga agaaaaagca gctgttgaat taga                      4784
<210> 25
   <211> 4936
   <212> DNA
   <213> Artificial Sequence
<220>
   <223> pOThy15
<400> 25
   gattaagtca tcttacctct tttattagtt ttttcttata atctaatgat aacattttta 60
   taattaatct ataaaccata tccctctttg gaatcaaaat ttattatcta ctcctttgta 120
   gatatgttat aatacaagta tcagatctgg gagaccacaa cggtttccca ctagaaataa 180
   ttttgtttaa ctttagaaag gagatatacg catgaaaaaa aagattatct cagctatttt 240
   aatgtctaca gtcatacttt ctgctgcagc cccgttgtca ggtgtttacg cctcagctgg   300
   tcaaggtact caatcagaaa actcatgtac tcactttcca ggtaacttgc caaacatgct   360
   tcgtgatttg cgtgatgctt tttcacgtgt taaaactttt tttcaaatga aagatcaact   420
   tgataacttg cttttgaaag aatcactttt ggaagatttt aaaggttacc ttggttgtca   480
   agctttgtca gaaatgatcc aattttacct tgaagaagtt atgccacaag ctgaaaacca   540
   agatccagat atcaaagctc acgttaactc attgggtgaa aaccttaaaa ctttgcgtct   600
   tcgtttgcgt cgttgtcacc gttttcttcc atgtgaaaac aaatcaaaag ctgttgaaca   660
   agttaaaaac gcttttaaca aattgcaaga aaaaggtatc tacaaagcta tgtcagaatt   720
   tgatatcttt atcaactaca tcgaagctta catgactatg aaaatccgta actaactaga   780
   attaatctat aagttactga caaaactgtc agtaactttt tttgtgggaa aaatgtattt   840
   ttatgaccgt aaagaatctg tcagtagaag tctgaaattc gtttaaaaat cgactagaat   900
   aggctttaac gacaagatgt tttaaagagt acgctctaaa tgtatttttg tatttttgtt   960
   tgattacgaa gtttaaattt aattgacaaa tgttttaaaa tgagtataat aggacttgta  1020
   accgatttta tttttataaa ggagaaagaa agatgaacaa acttttactt ggaacagcct  1080
   ttataggggc tagcttactg attggtgggg gtgctcatgc agatcaaatg tttatcgttt  1140
   gtataatcat aatactggtg agcactctat acaactagtg ggacaccaaa agaatgctaa  1200
   tgtaagtgcg ggttggactt atgaaggtgt cggttggatc gcaccaacaa caagttcaag  1260
   cccagtttac cgtgtgtaca atccaaatgc attattacac aaaaagcaag tatgaagccc  1320
   aaagtttagt aaataagggt tggaaatggg ataataacgg aaaggcggtc ttctattctg  1380
   gaggttctca agccgtatat gtcgcttata atcccaatgc acaatctggc gctcacaatt  1440
   acacggaaag tagctttgag caaaatagct tattgaatac tggttggaaa tatggggcag  1500
   tagcttggta cgggattgga gtaaaaaacg aaatgttaaa cattgctcaa attgttagtg  1560
   gtaatttttc tagtattgtt ggaacttgga aagatacttc tggaaatatg cttgaaatta  1620
   atgcaatggg aaatcttact ttaatatgga aaggggcaaa gaatcaaacc tttgaacttg  1680
   gcgcaggtca acaatttaat ggaactgcag atattgcctt aaaaaatgga gagatttccc  1740
   ctggtagtcc acttaacatt tttgttgtac caacagaagt gaattcactg gccgtcgttt  1800
   tacaacgtcg tgactgggaa aaccctggcg ttacccaact taatcgcctt gcagcacatc  1860
   cccctttcgc cagctggcgt aatagcgaag aggcccgcac cgatcgccct tcccaacagt   1920
   tgcgcagcct gaatggcgaa tggcgcctga tgcggtattt tctccttacg catctgtgcg  1980
   gtatttcaca ccgcatatgg tgcactagaa ctagcgattc tgaaatcacc atttaaaaaa  2040
   ctccaatcaa ataattttat aaagttagtg tatcactttg taatcataaa aacaacaata  2100
   aagctactta aatatagatt tataaaaaac gttggcgaaa acgttggcga ttcgttggcg  2160
   attgaaaaac cccttaaacc cttgagccag ttgggataga gcgtttttgg cacaaaaatt  2220
   ggcactcggc acttaatggg gggtcgtagt acggaagcaa aattcgcttc ctttcccccc  2280
   atttttttcc aaattccaaa tttttttcaa aaattttcca gcgctaccgc tcggcaaaat 2340
   tgcaagcaat ttttaaaatc aaacccatga gggaatttca ttccctcata ctcccttgag 2400
   cctcctccaa ccgaaataga agggcgctgc gcttattatt tcattcagtc atcggctttc 2460
   ataatctaac agacaacatc ttcgctgcaa agccacgcta cgctcaaggg cttttacgct 2520
   acgataacgc ctgttttaac gattatgccg ataactaaac gaaataaacg ctaaaacgtc 2580
   tcagaaacga ttttgagacg ttttaataaa aaatcgctag tccgaggcct cgacccgatt 2640
   cacaaaaaat aggcacacga aaaacaagtt aagggatgca gtttatgcat cccttaactt 2700
   acttattaaa taatttatag ctattgaaaa gagataagaa ttgttcaaag ctaatattgt 2760
   ttaaatcgtc aattcctgca tgttttaagg aattgttaaa ttgatttttt gtaaatattt 2820
   tcttgtattc tttgttaacc catttcataa cgaaataatt atacttttgt ttatctttgt 2880
   gtgatattct tgattttttt ctacttaatc tgataagtga gctattcact ttaggtttag 2940
   gatgaaaata ttctcttgga accatactta atatagaaat atcaacttct gccattaaaa 3000
   gtaatgccaa tgagcgtttt gtatttaata atcttttagc aaacccgtat tccacgatta 3060
   aataaatctc attagctata ctatcaaaaa caattttgcg tattatatcc gtacttatgt 3120
   tataaggtat attaccatat attttatagg attggttttt aggaaattta aactgcaata 3180
   tatccttgtt taaaacttgg aaattatcgt gatcaacaag tttattttct gtagttttgc 3240
   ataatttatg gtctatttca atggcagtta cgaaattaca cctctttact aattcaaggg 3300
   taaaatggcc ttttcctgag ccgatttcaa agatattatc atgttcattt aatcttatat 3360
   ttgtcattat tttatctata ttatgttttg aagtaataaa gttttgactg tgttttatat 3420
   ttttctcgtt cattataacc ctctttaatt tggttatatg aattttgctt attaacgatt 3480
   cattataacc acttattttt tgtttggttg ataatgaact gtgctgatta caaaaatact 3540
   aaaaatgccc atattttttc ctccttataa aattagtata attatagcac gggtcgagat 3600
   ccatgttctt tcctgcgtta tcccctgatt ctgtggataa ccgtattacc gcctttgagt 3660
   gagctgatac cgctcgccgc agccgaacga ccgagcgcag cgagtcagtg agcgaggaag 3720
   cggaagagcg cccaatacgc aaaccgcctc tccccgcgcg ttggccgatt cattaatgca 3780
   gctggcacga caggtttccc gactggaaag cgggcagtga gcgcaacgca attaatgtga 3840
   gttagctcac tcattaggca ccccaggctt tacactttat gcttccggct cgtatgttgt 3900
   gtggaattgt gagcggataa caatttcaca caggaaacag ctatgaccat gattacgcca 3960
   agcttgcatg cctgcaggtc gactctagag gatcctatat acaattgagc aaaagaaatt 4020
   tagttattaa attaccagct ggagttcctc caatggttgt agattcacta agtccagcaa 4080
   ttatttcaat ggtgattttc tgtttgatgt tcgggattcg tgtgggattc tcttatacgc 4140
   cattccatga tattttcaat ttctcaacac aactaattca agcaccgttg actggtgctg 4200
   tggcaaatcc atgggttctt atgggcatct ttacctttgg taatttctta tggttctttg 4260
   gtatccaccc taatttaatt gggggaattt taaatccatt gttattaaca atgtcatatg 4320
   ctaatattga tgcctatgct gccggaaaac ctgtaccata cttacaaatg atgattgtgt 4380
   ttgctgtggg tgcgaacgca tggggcggaa gtggaaatac ttatgggtta gttatttcaa 4440
   tgtttacggc aaaatctgaa cgctataaac aattattaaa attaggtgca attcctagta 4500
   ttttcaatat cagtgaacca ttactttttg gtcttccaat gatgttaaat cctcttttct 4560
   ttattccttt ggttttccaa ccagcaattt taggaactgt agcattgggc ttggcaaaga 4620
   tattatatat tacaaatctg aatccaatga cggcacttct tccttggacg acaccagcac 4680
   ctgtgagaat ggccatttca ggtggacttc catttttgat tatttttgca atctgtttag 4740
   tcttgaatgt tcttatttac tacccattct ttaaggtggc gtataataaa gctttagaag 4800
   aagaaaaagc agctgttgaa ttagagggtt cagaaactgc ctgatggata ttttttataa 4860
   atctggtttg aacaaattat attgacatct ctttttctat cctgataatt ctgagaggtt 4920
   attttgggaa atacta 4936
<210> 26
   <211> 4995
   <212> DNA
   <213> Artificial Sequence
<220>
   <223> pOThy16
<400> 26
   aatttcgatt aagtcatctt acctctttta ttagtttttt cttataatct aatgataaca     60
   tttttataat taatctataa accatatccc tctttggaat caaaatttat tatctactcc    120
   tttgtagata tgttataata caagtatcag atctgggaga ccacaacggt ttcccactag    180
   aaataatttt gtttaacttt agaaaggaga tatacgcatg aaaaaaaaga ttatctcagc    240
   tattttaatg tctacagtca tactttctgc tgcagccccg ttgtcaggtg tttacgcctc    300
   agctggtcaa ggtactcaat cagaaaactc atgtactcac tttccaggta acttgccaaa    360
   catgcttcgt gatttgcgtg atgctttttc acgtgttaaa actttttttc aaatgaaaga    420
   tcaacttgat aacttgcttt tgaaagaatc acttttggaa gattttaaag gttaccttgg    480
   ttgtcaagct ttgtcagaaa tgatccaatt ttaccttgaa gaagttatgc cacaagctga    540
   aaaccaagat ccagatatca aagctcacgt taactcattg ggtgaaaacc ttaaaacttt    600
   gcgtcttcgt ttgcgtcgtt gtcaccgttt tcttccatgt gaaaacaaat caaaagctgt    660
   tgaacaagtt aaaaacgctt ttaacaaatt gcaagaaaaa ggtatctaca aagctatgtc    720
   agaatttgat atctttatca actacatcga agcttacatg actatgaaaa tccgtaacta    780
   actagaatta atctataagt tactgacaaa actgtcagta actttttttg tgggaaaaat    840
   gtatttttat gaccgtaaag aatctgtcag tagaagtctg aaattcgttt aaaaatcgac    900
   tagaataggc tttaacgaca agatgtttta aagagtacgc tctaaatgta tttttgtatt    960
   tttgtttgat tacgaagttt aaatttaatt gacaaatgtt ttaaaatgag tataatagga   1020
   cttgtaaccg attttatttt tataaaggag aaagaaagat gaacaaactt ttacttggaa   1080
   cagcctttat aggggctagc ttactgattg gtgggggtgc tcatgcagat caaatgttta 1140
   tcgtttgtat aatcataata ctggtgagca ctctatacaa ctagtgggac accaaaagaa 1200
   tgctaatgta agtgcgggtt ggacttatga aggtgtcggt tggatcgcac caacaacaag 1260
   ttcaagccca gtttaccgtg tgtacaatcc aaatgcatta ttacacaaaa agcaagtatg 1320
   aagcccaaag tttagtaaat aagggttgga aatgggataa taacggaaag gcggtcttct 1380
   attctggagg ttctcaagcc gtatatgtcg cttataatcc caatgcacaa tctggcgctc 1440
   acaattacac ggaaagtagc tttgagcaaa atagcttatt gaatactggt tggaaatatg 1500
   gggcagtagc ttggtacggg attggagtaa aaaacgaaat gttaaacatt gctcaaattg 1560
   ttagtggtaa tttttctagt attgttggaa cttggaaaga tacttctgga aatatgcttg 1620
   aaattaatgc aatgggaaat cttactttaa tatggaaagg ggcaaagaat caaacctttg 1680
   aacttggcgc aggtcaacaa tttaatggaa ctgcagatat tgccttaaaa aatggagaga 1740
   tttcccctgg tagtccactt aacatttttg ttgtaccaac agaagtgaat tcactggccg 1800
   tcgttttaca acgtcgtgac tgggaaaacc ctggcgttac ccaacttaat cgccttgcag 1860
   cacatccccc tttcgccagc tggcgtaata gcgaagaggc ccgcaccgat cgcccttccc 1920
   aacagttgcg cagcctgaat ggcgaatggc gcctgatgcg gtattttctc cttacgcatc 1980
   tgtgcggtat ttcacaccgc atatggtgca ctagaactag cgattctgaa atcaccattt 2040
   aaaaaactcc aatcaaataa ttttataaag ttagtgtatc actttgtaat cataaaaaca 2100
   acaataaagc tacttaaata tagatttata aaaaacgttg gcgaaaacgt tggcgattcg 2160
   ttggcgattg aaaaacccct taaacccttg agccagttgg gatagagcgt ttttggcaca 2220
   aaaattggca ctcggcactt aatggggggt cgtagtacgg aagcaaaatt cgcttccttt 2280
   ccccccattt ttttccaaat tccaaatttt tttcaaaaat tttccagcgc taccgctcgg 2340
   caaaattgca agcaattttt aaaatcaaac ccatgaggga atttcattcc ctcatactcc 2400
   cttgagcctc ctccaaccga aatagaaggg cgctgcgctt attatttcat tcagtcatcg 2460
   gctttcataa tctaacagac aacatcttcg ctgcaaagcc acgctacgct caagggcttt 2520
   tacgctacga taacgcctgt tttaacgatt atgccgataa ctaaacgaaa taaacgctaa 2580
   aacgtctcag aaacgatttt gagacgtttt aataaaaaat cgctagtccg aggcctcgac 2640
   ccgattcaca aaaaataggc acacgaaaaa caagttaagg gatgcagttt atgcatccct 2700
   taacttactt attaaataat ttatagctat tgaaaagaga taagaattgt tcaaagctaa 2760
   tattgtttaa atcgtcaatt cctgcatgtt ttaaggaatt gttaaattga ttttttgtaa 2820
   atattttctt gtattctttg ttaacccatt tcataacgaa ataattatac ttttgtttat 2880
   ctttgtgtga tattcttgat ttttttctac ttaatctgat aagtgagcta ttcactttag 2940
   gtttaggatg aaaatattct cttggaacca tacttaatat agaaatatca acttctgcca 3000
   ttaaaagtaa tgccaatgag cgttttgtat ttaataatct tttagcaaac ccgtattcca 3060
   cgattaaata aatctcatta gctatactat caaaaacaat tttgcgtatt atatccgtac 3120
   ttatgttata aggtatatta ccatatattt tataggattg gtttttagga aatttaaact 3180
   gcaatatatc cttgtttaaa acttggaaat tatcgtgatc aacaagttta ttttctgtag 3240
   ttttgcataa tttatggtct atttcaatgg cagttacgaa attacacctc tttactaatt 3300
   caagggtaaa atggcctttt cctgagccga tttcaaagat attatcatgt tcatttaatc 3360
   ttatatttgt cattatttta tctatattat gttttgaagt aataaagttt tgactgtgtt 3420
   ttatattttt ctcgttcatt ataaccctct ttaatttggt tatatgaatt ttgcttatta 3480
   acgattcatt ataaccactt attttttgtt tggttgataa tgaactgtgc tgattacaaa 3540
   aatactaaaa atgcccatat tttttcctcc ttataaaatt agtataatta tagcacgggt 3600
   cgagatccat gttctttcct gcgttatccc ctgattctgt ggataaccgt attaccgcct 3660
   ttgagtgagc tgataccgct cgccgcagcc gaacgaccga gcgcagcgag tcagtgagcg 3720
   aggaagcgga agagcgccca atacgcaaac cgcctctccc cgcgcgttgg ccgattcatt 3780
   aatgcagctg gcacgacagg tttcccgact ggaaagcggg cagtgagcgc aacgcaatta 3840
   atgtgagtta gctcactcat taggcacccc aggctttaca ctttatgctt ccggctcgta 3900
   tgttgtgtgg aattgtgagc ggataacaat ttcacacagg aaacagctat gaccatgatt 3960
   acgccaagct tgcatgcctg caggtcgact ctagaggatc ctatatacaa ttgagcaaaa 4020
   gaaatttagt tattaaatta ccagctggag ttcctccaat ggttgtagat tcactaagtc 4080
   cagcaattat ttcaatggtg attttctgtt tgatgttcgg gattcgtgtg ggattctctt 4140
   atacgccatt ccatgatatt ttcaatttct caacacaact aattcaagca ccgttgactg 4200
   gtgctgtggc aaatccatgg gttcttatgg gcatctttac ctttggtaat ttcttatggt 4260
   tctttggtat ccaccctaat ttaattgggg gaattttaaa tccattgtta ttaacaatgt 4320
   catatgctaa tattgatgcc tatgctgccg gaaaacctgt accatactta caaatgatga 4380
   ttgtgtttgc tgtgggtgcg aacgcatggg gcggaagtgg aaatacttat gggttagtta 4440
   tttcaatgtt tacggcaaaa tctgaacgct ataaacaatt attaaaatta ggtgcaattc 4500
   ctagtatttt caatatcagt gaaccattac tttttggtct tccaatgatg ttaaatcctc 4560
   ttttctttat tcctttggtt ttccaaccag caattttagg aactgtagca ttgggcttgg 4620
   caaagatatt atatattaca aatctgaatc caatgacggc acttcttcct tggacgacac 4680
   cagcacctgt gagaatggcc atttcaggtg gacttccatt tttgattatt tttgcaatct 4740
   gtttagtctt gaatgttctt atttactacc cattctttaa ggtggcgtat aataaagctt 4800
   tagaagaaga aaaagcagct gttgaattag agggttcaga aactgcctga tggatatttt 4860
   ttataaatct ggtttgaaca aattatattg acatctcttt ttctatcctg ataattctga 4920
   gaggttattt tgggaaatac tattgaacca tatcgaggtg tgtggtataa tgaagggaat 4980
   taaaaaagat aggaa 4995

## Description

### Field of the invention

The invention relates to a recombinant *Lactococcus* strain, with environmentally limited growth and viability. More particularly, it relates to a recombinant *Lactococcus* that can only survive in a medium, where well-defined medium compounds are present. A preferred embodiment is a *Lactococcus* that may only survive in a host organism, where said medium compounds are present, but cannot survive outside the host organism in absence of said medium compounds. Moreover, said *Lactococcus* can be transformed with prophylactic and/or therapeutic molecules and can, as such, be used to treat diseases such as inflammatory bowel diseases.

### Background of the invention

Lactic acid bacteria have long time been used in a wide variety of industrial fermentation processes. They have generally-regarded-as-safe status, making them potentially useful organisms for the production of commercially important proteins. Indeed, several heterologous proteins, such as Interleukin-2, have been successfully produced in *Lactococcus* spp (Steidler *et* a/., 1995). It is, however, unwanted that such genetically modified micro organisms are surviving and spreading in the environment. To avoid unintentional release of genetically modified microorganisms, special guidelines for safe handling and technical requirements for physical containment are used. Although this may be useful in industrial fermentations, the physical containment is generally not considered as sufficient, and additional biological containment measures are taken to reduce the possibility of survival of the genetically modified microorganism in the environment. Biological containment is extremely important in cases where physical containment is difficult or even not applicable. This is, amongst others, the case in applications where genetically modified microorganisms are used as live vaccines or as vehicle for delivery of therapeutic compounds. Such applications have been described e.g. in WO 97/14806, which discloses the delivery of biologically active peptides, such as cytokines, to a subject, by recombinant non-invasive or non-pathogenic bacteria. WO 96/11277 describes the delivery of therapeutic compounds to an animal ― including humans - by administration of a recombinant bacterium, encoding the therapeutic protein. Steidler *et al*. (2000) describe the treatment of colitis by administration of a recombinant *Lactococcus lactis*, secreting interleukin-10. Such a delivery may indeed be extremely useful to treat a disease in an affected human or animal, but the recombinant bacterium may act as a harmful and pathogenic micro organism when it enters a non-affected subject, and an efficient biological containment that avoids such unintentional spreading of the micro organism is needed.

Biological containment systems for host organisms may be passive, based on a strict requirement of the host for specific growth factor or a nutrient, that is not present or present in low concentrations in the outside environment, or active, based on so-called suicidal genetic elements in the host, whereby the host is killed in the outside environment by a cell killing function, encoded by a gene that is under control of a promoter only being expressed under specific environmental conditions.

Passive biological containment systems are well known in microorganisms such as *Escherichia coli* or *Saccharomyces cerevisiae*. Such *E. coli* strains are disclosed e.g. in US4190495. WO 95/1061 discloses lactic acid bacterial suppressor mutants and their use as means of containment in lactic acid bacteria, but in that case, the containment is on the level of the plasmid, rather than on the level of the host strain and it stabilizes the plasmid in the host strain, but doesn't provide containment for the genetically modified host strain itself.

Active suicidal systems have been described by several authors. Such system consists of two elements: a lethal gene, and a control sequence that switches on the expression of the lethal gene under non-permissive conditions. WO 95/10614 discloses the use of a cytoplasmatically active truncated and/or mutated *Staphylococcus aureus* nuclease as lethal gene. WO 96/40947 discloses a recombinant bacterial system with environmentally limited viability, based on the expression of either an essential gene, expressed when the cell is in the permissive environment and is not expressed or temporarily expressed when the cell is in the non-permissive environment and/or a lethal gene, wherein expression of the gene is lethal to the cell and the lethal gene is expressed when the cell is in the non-permissive environment but not when the cell is in the permissive environment. WO 99/58652 describes a biological containment system based on the relE cytotoxin. However, most systems have been elaborated for *Escherichia coli* (Tedkin *et al.,* 1995; Knudsen *et al*., 1995; Schweder *et al.,* 1995) or for *Pseudomonas* (Kaplan *et al.,* 1999; Molino *et al*., 1998). Although several of the containment systems theoretically can by applied to lactic acid bacteria, no specific biological containment system for *Lactococcus* has been described that allows the usage of a self-containing and transformed *Lactococcus* to deliver prophylactic and/or therapeutic molecules in order to prevent and/or treat diseases.

### Brief description of the figures

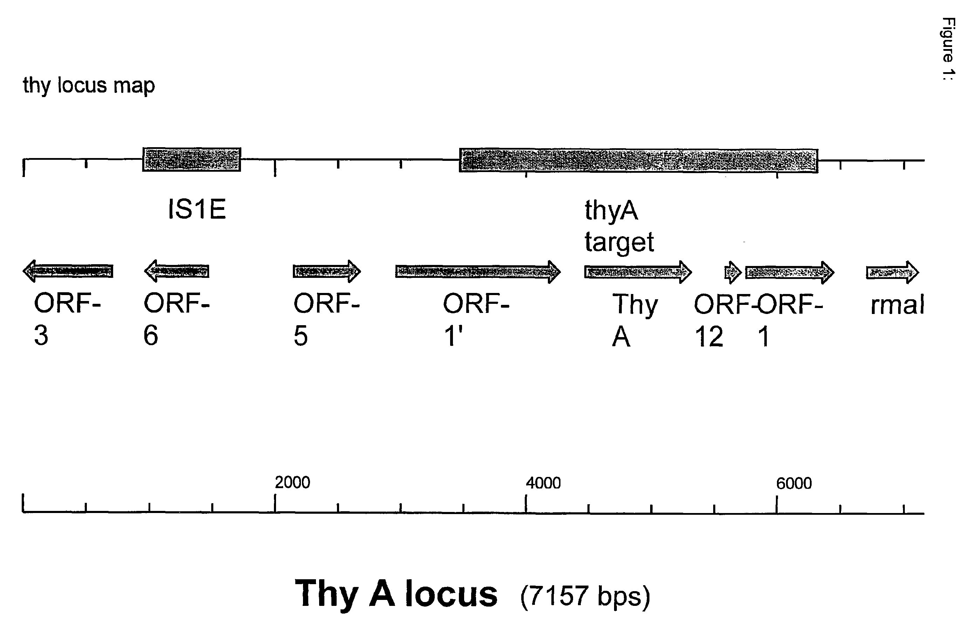

**Figure 1:** Map of the MG1363 *thyA* locus
**Figure 2:** Schematic representation of the different expression modules as present on pOThy plasmids ands genomic integrants of hIL-10. Black parts represent original *L. lactis* MG1363 genetic information, white parts represent recombinant genetic information.

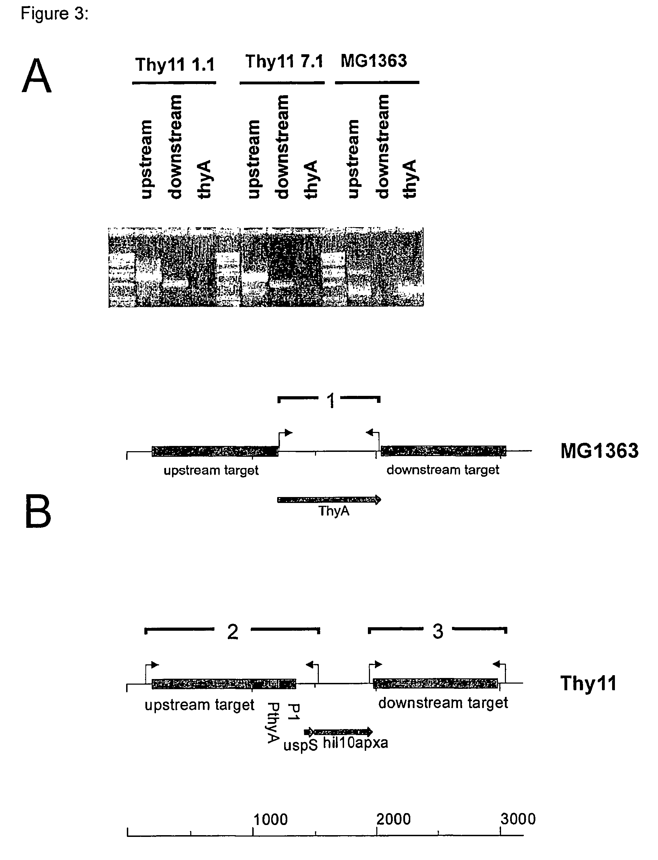

**Figure 3:** PCR identification of Thy11 (Thy11 1.1 and Thy11 7.1 represent individually obtained, identical clones). Standard PCR reactions were performed by using aliquots of saturated cultures of the indicated strains as a source of DNA template. Panel A shows an agarose gel of the products of the indicated PCR reactions. Panel B shows the positions at which primers attach in the thyA (1), upstream (2) or downstream (3) PCR's. Oligonucleotide primers used: (1): ATgACTTACgCAgATCAAgTTTTT and TTAAATTgCTAAATCAAATTTCAATTg (2): TCTgATTgAgTACCTTgACC and gCAATCATAATTggTTTTATTg (3): CTTACATgACTATgAAAATCCg and cTTTTTTATTATTAgggAAAgCA.
**Figure 4:** PCR identification of Thy11, Thy12, Thy15 and Thy16. Standard PCR reactions were performed by using three days old colonies of the indicated strains as a source of DNA template.
   Panel A shows the positions at which primers attach in the upstream (1), downstream (2) or thyA (3), PCR's. Oligonucleotide primers used: (1): ATgACTTACgCAgATCAAgTTTTT and TTAAATTgCTAAATCAAATTTCAATTg (2): TCTgATTgAgTACCTTgACC and gCAATCATAATTggTTTTATTg (3): CTTACATgACTATgAAAATCCg and cTTTTTTATTATTAgggAAAgCA
   Panel B shows an agarose gel of the products of the indicated PCR reactions.

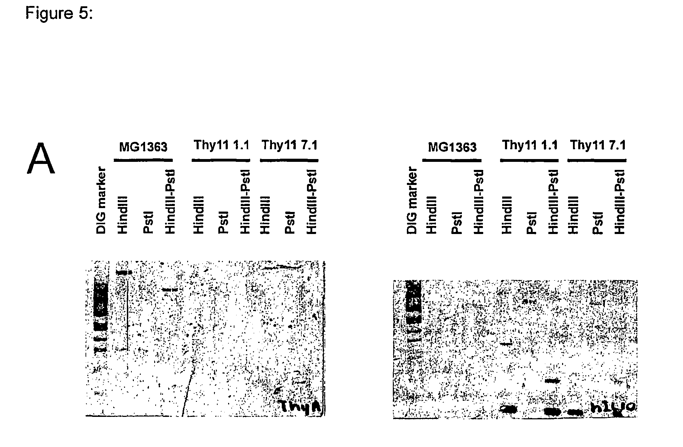

**Figure 5:** Southern blot analysis of the indicated strains. Chromosomal DNA was extracted and digested with the indicated restriction enzymes. Following agarose gel electrophoresis the DNA was transferred to a membrane and the chromosome structure around the thyA locus was revealed by use of DIG labelled thyA or hIL-10 DNA fragments (panel A). Panel B shows a schematic overview of the predicted structure of the thyA locus in both MG1363 and Thy11.

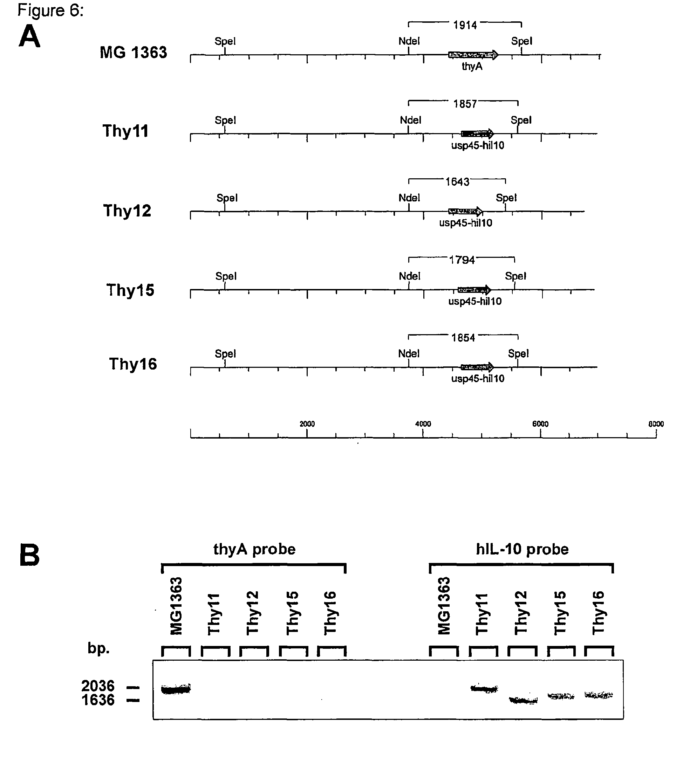

**Figure 6:** Panel A shows a schematic overview of part of the predicted structure of the *L. lactis* chromosome at the *thy*A locus in MG1363, Thy11, Thy12, Thy15 and Thy16. Numbers indicate base pairs
   Panel B. Southern blot analysis of the indicated strains. Chromosomal DNA was extracted and digested with Ndel and Spel restriction enzymes. Following agarose gel electrophoresis the DNA was transferred to a membrane and the chromosome structure around the thyA locus was revealed by use of DIG labelled thyA or hIL-10 DNA fragments.

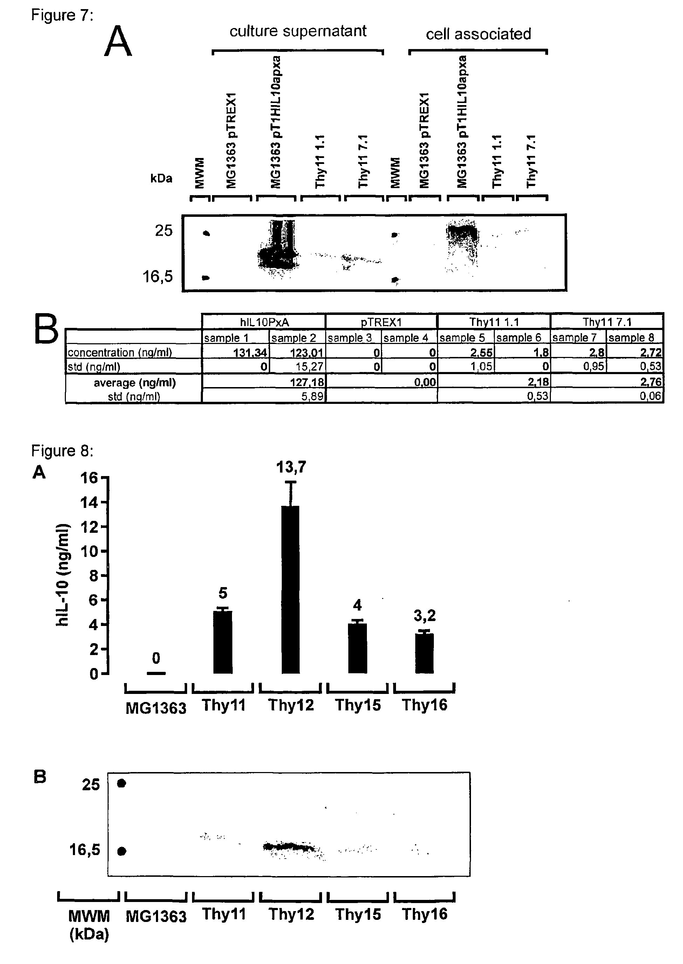

**Figure 7:** Production of hIL-10. Panel A shows a western blot revealed with anti-hIL-10 antiserum of culture supernatant and cell associated proteins of the indicated strains. Panel B shows quantification (by ELISA) of hIL-10 present in the culture supernatant.
**Figure 8:** Production of hIL-10. Panel A shows quantification (by ELISA) of hIL-10 present in the culture supernatant of the indicated strains. Panel B shows a western blot revealed with anti-hIL-10 antiserum of culture supernatant proteins of the indicated strains.

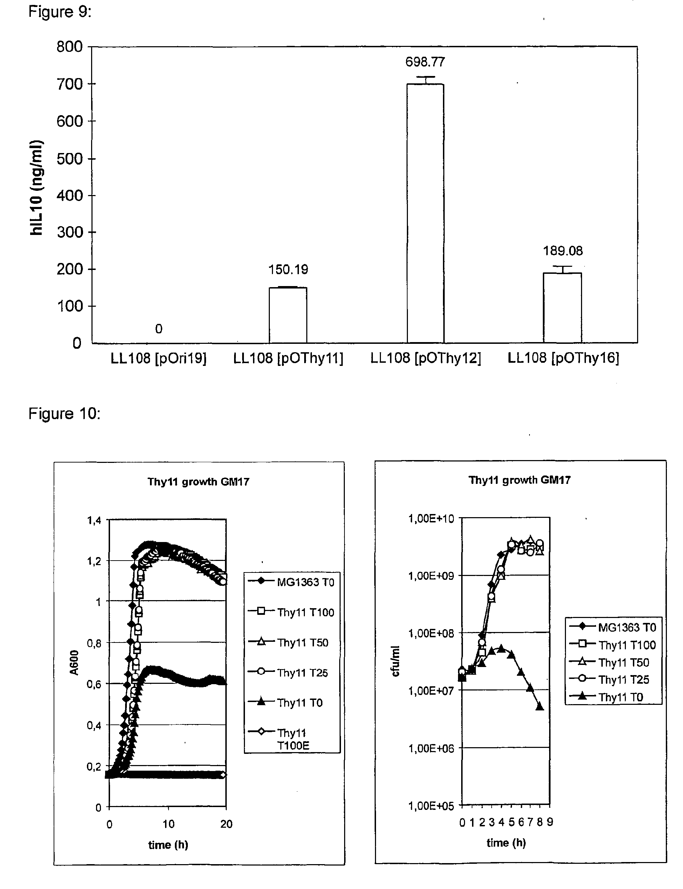

**Figure 9:** Production of hIL-10 by the *L. lactis* strains LL108 carrying either pOThy11, pOThy12, or pOThy16. Quantification (by ELISA) of hIL-10 present in the culture supernatant of the indicated strains. The N-terminal protein sequence of the recombinant hIL-10 was determined by Edman degradation and was shown identical to the structure as predicted for the mature, recombinant hIL-10. The protein showed full biological activity.
**Figure 10:** Growth rate of the indicated strains in GM17 containing 100µg/ml (T100) 50µg/ml (T50) 25µg/ml (T25) or no (T0) extra thymidine and possibly supplemented with 5µg/ml of erythromycin (E). Saturated overnight cultures (prepared in T50) were diluted 1:100 in the indicated culture media. Panel A shows the kinetics of absorbance accumulation. Panel B shows the kinetics of the number of colony forming units (cfu)
per ml of culture.

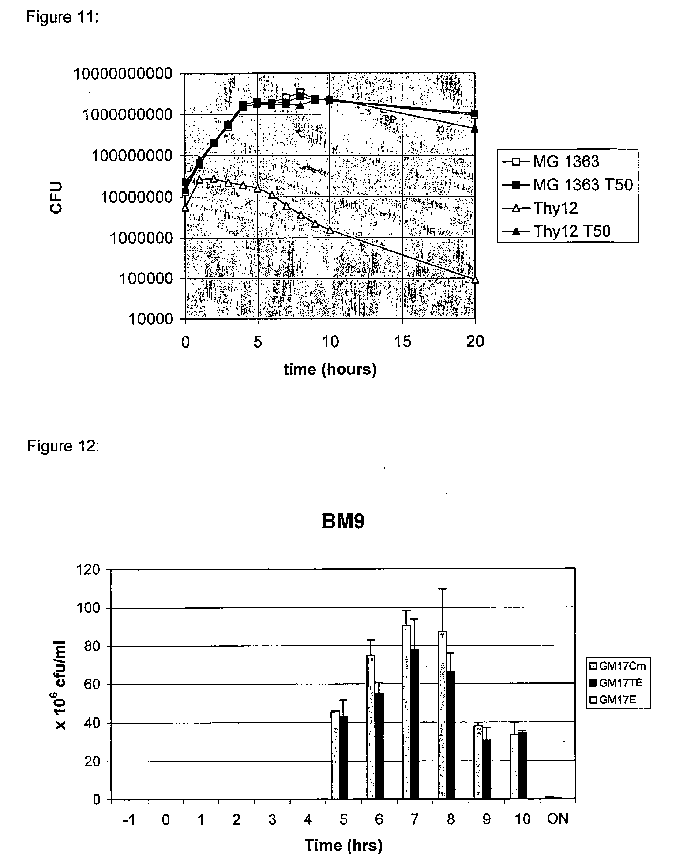

**Figure 11:** Growth rate of MG1363 and Thy12 in thymidine free medium (TFM). TFM was prepared by growing *L. lactis* Thy12 bacteria in GM17, removing the bacteria by subsequent centrifugation and filtration on a 0,22µm pore size filter, adjusting the pH to 7,0 and autoclaving.
   MG1363 and Thy12 bacteria were collected from an overnight culture in GM17 or GM17+50µg/ml of thymidine respectively and washed in M9 buffer (6 g/l Na₂HPO₄, 3 g/l KH₂PO₄, 1 g/l NH₄Cl, 0,5 g/l NaCl in water). The suspensions of both were either diluted in TFM or TFM supplemented with 50µg/ml of thymidine (T50). CFU counts were determined at different time points: t=0, 1, 2, 3, 4, 5, 6, 7, 8, 9, 10 and 20 hours.
   This shows that Thy12 viability is severely impaired in the absence of thymidine.
**Figure 12:** Intestinal passage and viability: ***L.** lactis* MG1363 was transformed with the plasmid pLET2N which carries a chloramphenicol (Cm) resistance marker. L. lactis Thy12 was transformed with the plasmid pT1NX which carries an erythromycin (Em) resistance marker. Of both strains 10⁹ bacteria were resuspended in BM9 (6 g/l Na₂HPO₄, 3 g/l KH₂PO₄, 1 g/l NH₄Cl, 0,5 g/l NaCl in 25 mM NaHCO₃ + 25 mM Na₂CO₃), mixed and inoculated in three mice at t=0h. Faeces were collected of the time intervals -1 to 0, 0 to 1, 1 to 2, 2 to 3, 3 to 4, 4 to 5, 5 to 6, 6 to 7, 7 to 8, 8 to 9, 9 to 10 and 10 to overnight. All samples were resuspended in isotonic buffer and appropriate dilutions were plated on GM17 (M17 medium, Difco, St.Louis supplemented with 0,5% glucose) plates containing either Cm, Em or Em+ 50µg/ml thymidine. Colony forming units for the different plates are represented in the graph.

### Description of the invention

It is the objective of the present invention to provide a suitable biological containment system for *Lactococcus.*

A first aspect of the invention is the use of a strain of *Lactococcus* sp. comprising a thymidylate synthase gene inactivated by gene disruption. Even more preferably, said Lactococcus sp. is *Lactococcus lactis*. A special embodiment is a *Lactococcus* sp. strain, preferably *Lactococcus lactis*, more preferably a *Lactococcus lactis* MG1363 derivative, whereby the thymidylate synthase gene has been disrupted and replaced by an interleukin-10 expression unit. Said interleukin-10 expression unit is preferably, but not limited to, a human interleukin-10 expression unit or gene encoding for human interleukin-10.

Another aspect of the invention is the use of a strain according to the invention as host strain for transformation, whereby the transforming plasmid does not comprise an thymidylate synthase gene inactivated by gene disruption. Still another aspect of the invention is a transformed strain of *Lactococcus* sp. according to the invention, comprising a plasmid that comprises a thymidylate synthase gene inactivated by gene disruption. Another aspect of the invention relates to a transformed strain of *Lactococcus* sp. comprising a gene or expression unit encoding a prophylactic and/or therapeutic molecule such as interleukin-10. Consequently, the present invention also relates to the usage of a transformed strain of *Lactococcus* sp. to deliver prophylactic and/or therapeutic molecules, and as such, to treat diseases. Methods to deliver said molecules and methods to treat diseases such as inflammatory bowel diseases are explained in detail in WO 97/14806 and WO 00/23471 to Steidler et al. and in Steidler et al. (Science 2000, 289:1352).

Another aspect of the invention is a medical preparation, comprising a transformed strain of *Lactococcus* sp., according to the invention.

The *Lactococcus lactis* subsp. *lactis* thymidylate synthase gene (*thyA*) has been cloned by Ross *et al*. (1990a); it sequence is comprised in SEQ ID N° 3 and SEQ ID N° 5. EP0406003 discloses a vector devoid of antibiotic resistance and bearing a thymidylate synthase gene as a selection marker; the same vector has been described by Ross *et al*. (1990b). However, this vector could not be used in a *Lactococcus lactis* strain due to the lack of a suitable *thyA* mutant that had never been described. The present invention discloses how to construct such mutant by gene disruption, using homologous recombination in *Lactococcus.* In a preferred embodiment, the *thyA* gene is disrupted by a functional human interleukin-10 expression cassette. However, it is clear that any construct can be used for gene disruption, as long as it results in an inactivation of the *thyA* gene or in an inactive thymidylate synthase. As a non-limiting example, the homologous recombination may result in a deletion of the gene, in one or more amino acid substitutions that lead to an inactive form of the thymidylate synthase, or to a frameshift mutation resulting in a truncated form of the protein.

Such a *Lactococcus* sp. *thyA* mutant is very useful as a host strain for transformation, in situations where more severe containment than purely physical containment is needed. Indeed, *thyA* mutants cannot survive in an environment without, or with only a limited concentration of thymidine and/or thymine. When such a strain is transformed with a plasmid that doesn't comprise an intact *thyA* gene and cannot complement the mutation, the transformed strain will become suicidal in a thymidine/thymine poor environment. Such a strain can be used in a fermentor, as an additional protection for the physical containment. Moreover, the present invention discloses that such a strain is especially useful in cases where the strain is used as a delivery vehicle in an animal body. Indeed, when such a transformed strain is given for example orally to an animal - including humans - it survives in the gut, provided a sufficiently high concentration of thymidine/thymine is present, and produces homologous and/or heterologous proteins, such as human interleukin-10, that may be beneficial for said animal. The present invention further demonstrates that the transformed strains surprisingly pass the gut at the same speed as the control strains and shows that their loss of viability is indeed not different from that of the control strains. However, once said strain is secreted in the environment, e.g. in the faeces, it is not able to survive any longer.

The transforming plasmid can be any plasmid, as long as it cannot complement the *thyA* mutation. It may be a selfreplicating plasmid that preferably carries one or more genes of interest and one or more resistance markers, or it may be an integrative plasmid. In the latter case, the integrative plasmid itself may be used to create the mutation, by causing integration at the *thyA* site, whereby the *thyA* gene is inactivated. Preferably, the active *thyA* gene is replaced by double homologous recombination by a cassette comprising the gene or genes of interest, flanked by targeting sequences that target the insertion to the *thyA* target site. It is of extreme importance that these sequences are sufficiently long and sufficiently homologous to obtain to integrate the sequence into the target site. Preferably, said targeting sequences consist of at least 100 contiguous nucleotides of SEQ ID N°1 at one side of the gene of interest, and at least 100 contiguous nucleotides of SEQ ID N°2 at the other side; more preferably, said targeting sequences consists of at least 500 contiguous nucleotides of SEQ ID N°1 at one side of the gene of interest, and at least 500 contiguous nucleotides of the SEQ ID N° 2 at the other side; most preferably, said targeting sequences consists of SEQ ID N°1 at one side of the gene of interest and SEQ ID N°2 at the other side, or said targeting sequences consist of at least 100 nucleotides that are at least 80% identical, preferably 90% identical to a region of SEQ ID N° 1 at one side of the gene of interest, and of at least 100 nucleotides that are at least 80% identical, preferably 90% identical to a region of SEQ ID N° 2 at the other side of the gene of interest, preferably said targeting sequences consist of at least 500 nucleotides that are at least 80% identical, preferably 90% identical to a region of SEQ ID N° 1 at one side of the gene of interest, and of at least 500 nucleotides that are at least 80% identical, preferably 90% identical to a region of SEQ ID N° 2 at the other side of the gene of interest, most preferably said targeting sequences consist of at least 1000 nucleotides that are at least 80% identical, preferably 90% identical to a region of SEQ ID N° 1 at one side of the gene of interest, and of at least 1000 nucleotides that are at least 80% identical, preferably 90% identical to a region of SEQ ID N° 2 at the other side of the gene of interest. The percentage identity is measured with BLAST, according to Altschul *et al*. (1997). A preferred example of a sequence, homologous to SEQ ID N°1 is given in SEQ ID N° 7. For the purpose of the invention, SEQ ID N° 1 and SEQ ID N° 7 are interchangeable.

Transformation methods of *Lactococcus* are known to the person skilled in the art, and include, but are not limited to protoplast transformation and electroporation.

A transformed *Lactococcus* sp. strain according to the invention is useful for the delivery of prophylactic and/or therapeutical molecules and can be used in a pharmaceutical composition. The delivery of such molecules has been disclosed, as a non-limiting example, in WO 97/14806 and in WO 98/31786. Prophylactic and/or therapeutical molecules include, but are not limited to polypetides such as insuline, growth hormone, prolactine, calcitonin, group 1 cytokines, group 2 cytokines and group 3 cytokines and polysaccharides such as polysaccharide antigens from pathogenic bacteria. A preferred embodiment is the use of a *Lactococcus* sp. strain according to the invention to deliver human interleukin-10. This strain can be used in the manufacture of a medicament to treat Crohn's disease as indicated above.

### Examples

From L. lactis MG1363 (Gasson, 1983) we have cloned out the regions flanking the sequence according to Ross *et al*. (1990a)

The knowledge of these sequences is of critical importance for the genetic engineering of any lactococcus strain in a way as described below, as the strategy will employ double homologous recombination in the areas 1000 bp at the 5'end (SEQ ID N°1) and 1000 bp at the 3'end (SEQ ID N°2) of thyA, the "thyA target". These sequences are not available from any public source to date. We have cloned these flanking DNA fragments and have identified their sequence. The sequence of the whole locus is shown in SEQ ID N°3; a mutant version of this sequence is shown in SEQ ID N°5. Both the 5' and 3' sequences are different from the sequence at genbank AE006385 describing the *L. lactis* IL1403 sequence (Bolotin, in press) or at AF336368 describing the *L. lactis* subsp. *lactis* CHCC373 sequence. From the literature it is obvious that homologous recombination by use of the published sequences adjacent to *thyA* (Ross *et al*., 1990a) (86 bp at the 5'end and 31 bp at the 3'end) is virtually impossible due to the shortness of the sequences. Indeed, Biswas *et al*. (1993) describe a logarithmically decreasing correlation between length of the homologous sequences and frequency of integration. The sequences of *L. lactis* Thy 11, Thy 12, Thy 15 and Thy 16 at the thyA locus as determined in the present invention are given by SEQ ID N° 19, 20, 21, 22 respectively.

The *thyA* replacement is performed by making suitable replacements in a plasmid borne version of the *thyA* target, as described below. The carrier plasmid is a derivative of pORI19 (Law *et al.,* 1995) a replication defective plasmid, which only transfers the erythromycin resistance to a given strain when a first homologous recombination, at either the 5' 1000bp or at the 3'1000bp of the *thyA* target. A second homologous recombination at the 3' 1000bp or at the 5' 1000bp of the *thyA* target yields the desired strain.

The *thyA* gene is replaced by a synthetic gene encoding a protein which has the *L. lactis* Usp45 secretion leader (van Asseldonk *et al.,* 1990) fused to a protein of identical amino acid sequence than: (a) the mature part of human-interleukin 10 (hIL-10) or (b) the mature part of hIL-10 in which proline at position 2 had been replaced with alanine or (c) the mature part of hIL-10 in which the first two amino acids had been deleted; (a), (b) and (c) are called hIL-10 analogs, the fusion products are called Usp45-hIL-10.

The *thyA* gene is replaced by an expression unit comprising the lactococcal P1 promotor (Waterfield *et al.,* 1995), the *E. coli* bacteriophageT7 expression signals: putative RNA stabilising sequence and modified gene10 ribosomal binding site (Wells and Schofield, 1996).

At the 5' end the insertion is performed in such way that the ATG of thyA is fused to the P1-T7Usp45-hIL-10 expression unit.

Alternatively, at the 5' end the insertion is performed in such way that the thyA ATG is not included:

Alternatively, at the 5' end the insertion is performed in such way that the thyA promotor [Ross, 1990 a] is not included:

At the 3' end an ACTAGT Spel restriction site was engineered immediately adjacent to the TAA stop codon of the usp45-hIL-10 sequence. This was ligated in a TCTAGA *Xba*l restriction site, which was engineered immediately following the thyA stop codon

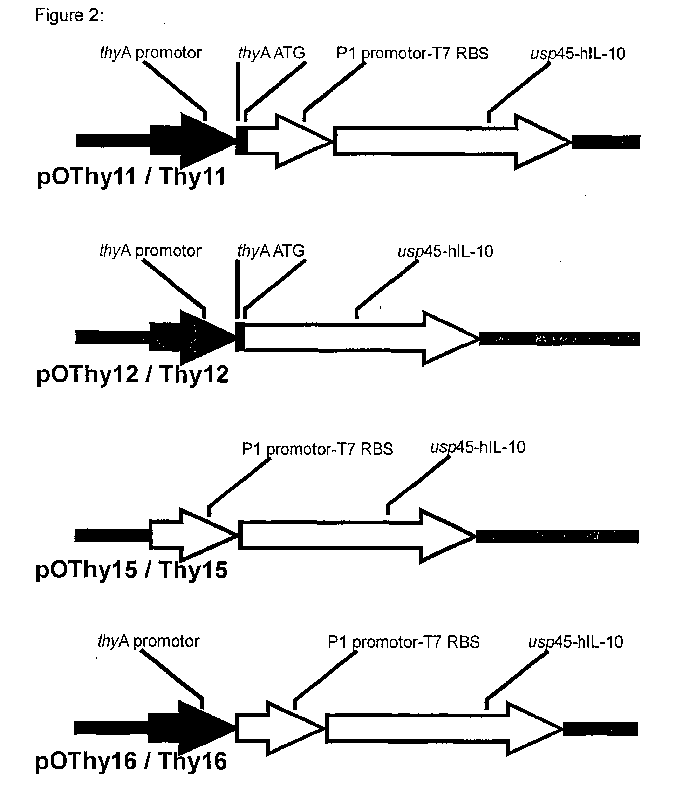

These constructs are depicted in figure 2. The sequences of pOThy11, pOThy12 pOThy15 and pOThy16 are given by SEQ ID N°'s 23, 24, 25, 26 respectively.

The resulting strains are *thyA* deficient, a mutant not yet described for *L. lactis.* It is strictly dependent upon the addition of thymine or thymidine for growth.

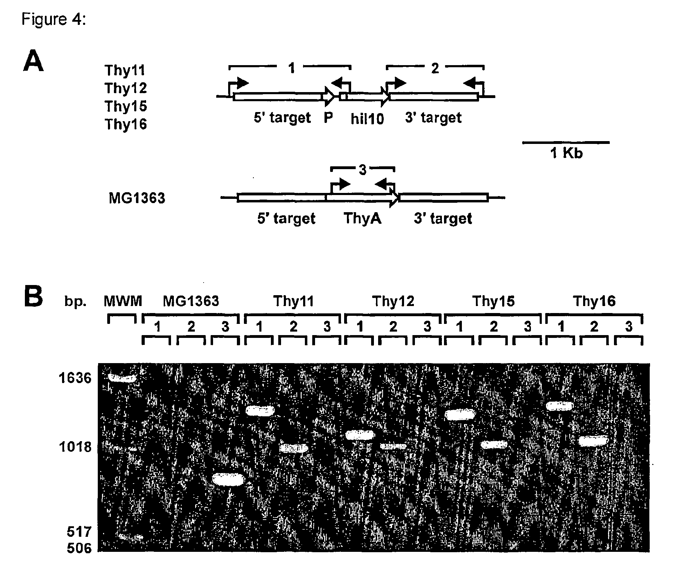

The map of the deletion, as well as the PCR analysis of all the isolates/mutants of the present invention are shown in figures 3 and 4. The presence of the thymidylate synthase and the interleukin 10 gene in the wild type strain and in the independent isolates/mutant was analyzed by Southern analysis as shown in figures 5 and 6. The region around the inserted hIL-10 gene was isolated by PCR and the DNA sequence was verified. The structure is identical to the predicted sequence.

Human interleukin 10 production in the mutants was checked by western blot analysis, and compared with the parental strain, transformed with pTREX1 as negative control, and the parental strain, transformed with the IL10 producing plasmid pT1HIL10apxa as positive control (figure 7A). The concentration in the culture supernatant was quantified using ELISA. As shown in figure 7B, both isolates of the mutant produce a comparable, significant amount of hIL-10, be it far less than the strain, transformed with the non-integrative plasmid pT1HIL10apxa. Figure 8 (panel A and B) further demonstrates that all mutants produce a significant amount of h-IL 10.

Figure 9 shows the production of hIL-10 by the *L. lactis* strains LL108 carrying either pOThy11, pOThy12, or pOThy16. Quantification (by ELISA) of hIL-10 present in the culture supernatant of the indicated strains. The N-terminal protein sequence of the recombinant hIL-10 was determined by Edman degradation and was shown identical to the structure as predicted for the mature, recombinant hIL-10. The protein showed full biological activity. LL108 is a *L. lactis* strain carrying a genomic integration of the repA gene, required for replication of pORI19 derived plasmids such as pOThy11, pOThy12, pOThy15 or pOThy16. This strain was kindly donated by dr. Jan Kok, University of Groningen. The plasmids pOThy11, pOThy12, pOThy15 and pOThy16 carry the synthetic human IL-10 gene in different promotor configurations (see Fig. 2), flanked by approximately 1 kB of genomic DNA derived from the thyA locus, upstream and downstream from thyA. These plasmids were used for the construction of the genomic integration as described.

The effect of the thymidilate synthase deletion on the growth in thymidine less and thymidine supplemented media was tested; the results are summarized in figures 10 and 11. Absence of thymidine in the medium strongly limits the growth of the mutant, and even results in a decrease of colony forming units after four hours of cultivation. Addition of thymidine to the medium results in an identical growth curve and amount of colony forming units, compared to the wild type strain, indicating that the mutant doesn't affect the growth or viability in thymidine supplemented medium. Fig. 11 clearly demonstrates that Thy12 viability is severely impaired in the absence of thymidine.

Fig. 12 finally shows that *L. lactis* Thy12 passes the intestine of the mice at the same speed as MG1363. Loss of viability does not appear different between Thy12 and MG1363. Thy12 appear fully dependent on thymidine for growth, indicating that no Thy12 bacteria had taken up a foreign thyA gene.

### References

- Altschul, S. F., Madden, T.L., Schäffer, A.A., Zhang, J., Zhang, Z., Miller, W. and Lipman D.J. (1997). Gapped BLAST and PSI-BLAST: a new generation of protein database search programs. *Nucleic Acids Res.* **25,** 3389 - 3402.
- Biswas, I., Gruss, A., Ehrlich, S.D. et al. (1993) High-efficiency gene inactivation and replacement system for gram- positive bacteria. J. *Bacteriol.* **175,** 3628 - 3635.
- Gasson, M. J. (1983). Plasmid complements of Streptococcus lactis NCDO 712 and other lactic streptococci after protoplast-induced curing. *J. Bacteriol*. **154**, 1 - 9.
- Kaplan, D.L., Mello, C., Sano, T., Cantor, C. and Smith, C. (1999). Streptavadin-based containment system for genetically engineered microorganisms. *Biomol. Eng.* **31**, 135 - 140.
- Knudsen, S., Saadbye, P., Hansen, L.H., Collier, A., Jacobsen, B.L., Schlundt, J. And Karlstrom, O.H. (1995). Development and testing of improved suicide functions for biological containment of bacteria. *Appl. Environ. Microbiol*. **61**, 985 - 991.
- Law, J., Buist, G., Haandrikman, A. et al. (1995). A system to generate chromosomal mutations in Lactococcus lactis which allows fast analysis of targeted genes. J. *Bacteriol*. 177, 7011 - 7018.
- Molina, L., Ramos, C., Ronchel, M.C., Molin, S. and Ramos, J.L. (1998). Construction of an efficient biologically contained pseudomonas putida strain and its survival in outdoor assays. *Appl. Environ. Microbiol*. **64**, 2072 - 2078.
- Ross, P., O'Gara, F. and Condon, S. (1990a). Cloning and characterization of the thymidylate synthase gene from *Lactococcus lactis* subsp. *Lactis. Appl. Environ*. *Microbiol.* **56**, 2156-2163.
- Ross, P., O'Gara, F. and Condon, S. (1990b). Thymidylate synthase gene from *Lactococcus lactis* as a genetic marker: an alternative to antibiotic resistance. *Appl. Environ. Microbiol.* **56,** 2164-2169.
- Schweder, T., Hofmann, K. And Hecker, M. (1995). *Escherichia coli* K12 relA strains as safe hosts for expression of recombinant DNA. *Appl. Environ. Microbiol.* **42,** 718-723.
- Steidler, L., Hans, W., Schotte, L., Neirynck, S., Obermeier, F., Falk, W., Fier, W. and Remaut, E. (2000). Treatment of murine colitis by *Lactococcus lactis* secreting Interleukin-10. *Science* **289**, 1352 - 1355.
- Steidler, L., Wells, J.M., Raeymaekers, A., Vandekerckhove, J., Fiers, W. And Remaut, E. (1995). Secretion of biologically active murine Interleukin-2 by *Lactococcus lactis* subsp. *Lactis. Appl. Environ. Microbiol.* **61,** 1627-1629.
- Tedin, K. Witte, A., Reisinger, G., Lubitz, W. and Basi, U. (1995). Evaluation of the *E. coli* ribosomal rrnB P1 promoter and phage derived lysis genes for the use in biological containment system: a concept study. *J. Biotechnol*. **39,** 137 - 148.
- van Asseldonk, M., Rutten, G., Oteman, M. et al. (1990). Cloning of usp45, a gene encoding a secreted protein from Lactococcus lactis subsp. lactis MG1363. *Gene* 95, 155 - 160.
- Waterfield, N.R., Le Page, R.W., Wilson, P.W. et al. (1995) The isolation of lactococcal promoters and their use in investigating bacterial luciferase synthesis in Lactococcus lactis. *Gene* **165**,9 - 15.
- Wells, J.M. and Schofield, K.M. (1996) Cloning and expression vectors for Lactococci. *Nato ASI series* H **98,** 37 - 62.

## Claims

1. The use of an isolated strain of *Lactococcus* sp., comprising a thymidylate synthase gene, inactivated by gene disruption, for the preparation of a medicament for the delivery of a prophylactic and/or therapeutic molecule.

2. The use of an isolated strain of *Lactococcus* sp, according to claim 1, whereby said thymidylate synthase gene is disrupted by the gene encoding said prophylactic and/or therapeutic molecule.

3. The use of an isolated strain of *Lactococcus* sp. according to claim 1 or 2, whereby said prophylactic and/or therapeutic molecule is interleukin 10.

4. The use of an isolated strain of *Lactococcus* sp. according to claim 3 for the preparation of a medicament to treat inflammatory bowel disease.

5. A pharmaceutical composition, comprising an isolated strain of *Lactococcus* sp., producing a prophylactic and /or therapeutic molecule, said strain comprising a thymidylate synthase gene, inactivated by gene disruption.

6. A pharmaceutical composition according to claim 4 whereby said thymidylate synthase gene is disrupted by the gene encoding said prophylactic and/or therapeutic molecule.

7. A pharmaceutical composition according to claim 4 or 5, whereby said prophylactic and/or therapeutic molearle is interleukin 10.

## Patentansprüche

1. Verwendung eines isolierten *Lactococcus sp*. Stamms, umfassend ein Thymidylat-Synthase-Gen, das mittels Gendisruption inaktiviert wurde für die Zubereitung eines Medikaments für die Zufuhr eines prophylaktischen und/oder therapeutischen Moleküls.

2. Die Verwendung eines isolierten *Lactococcus sp.* Stamms nach Anspruch 1, wobei das Thymidylat-Synthase-Gen durch das Gen, das für das prophylaktische und/oder therapeutische Molekül kodiert disrumpiert wird.

3. Die Verwendung eines isolierten *Lactococcus sp*. Stamms nach Anspruch 1 oder 2, wobei das prophylaktische und/oder therapeutische Molekül Interleukin 10 ist.

4. Die Verwendung eines isolierten *Lactococcus sp.* Stamms nach Anspruch 3 für die Zubereitung eines Medikaments zur Behandlung entzündlicher Darmerkrankung.

5. Eine pharmazeutische Zusammensetzung umfassend einen isolierten *Lactococcus sp.* Stamm, der ein prophylaktisches und/oder therapeutisches Molekül herstellt, wobei der Stamm ein Thymidylat-Synthase-Gen umfasst, das mittels Gendisruption inaktiviert ist.

6. Die pharmazeutische Zusammensetzung nach Anspruch 4, wobei das Thymidylat-Synthase-Gen durch das Gen, das für das prophylaktische und/oder therapeutische Molekül kodiert, disrumpiert wird.

7. Die pharmazeutische Zusammensetzung nach Anspruch 4 oder 5, wobei das prophylaktische und/oder therapeutische Molekül Interleukin 10 ist.

## Revendications

1. Utilisation d'une souche isolée de *Lactococcus* sp., comprenant un gène de thymidylate synthase, inactivé par rupture de gène, pour la préparation d'un médicament destiné à l'administration d'une molécule prophylactique et/ou thérapeutique.

2. Utilisation d'une souche isolée de *Lactococcus* sp. selon la revendication 1, dans laquelle ledit gène de thymidylate synthase est rompu par le gène codant ladite molécule prophylactique et/ou thérapeutique.

3. Utilisation d'une souche isolée de *Lactococcus* sp. selon la revendication 1 ou 2, dans laquelle ladite molécule prophylactique et/ou thérapeutique est l'interleukine 10.

4. Utilisation d'une souche isolée de *Lactococcus* sp. selon la revendication 3, pour la préparation d'un médicament destiné à traiter une affection abdominale inflammatoire.

5. Composition pharmaceutique, comprenant une souche isolée de *Lactococcus* sp., produisant une molécule prophylactique et/ou thérapeutique, ladite souche comprenant un gène de thymidylate synthase, inactivée par rupture de gène.

6. Composition pharmaceutique selon la revendication 4, dans laquelle ledit gène de thymidylate synthase est rompu par le gène codant ladite molécule prophylactique et/ou thérapeutique.

7. Composition pharmaceutique selon la revendication 4 ou 5, dans laquelle ladite molécule prophylactique et/ou thérapeutique est l'interlokine 10.
